# EUROPEAN PATENT SPECIFICATION

(11) **EP 2 195 655 B1**
(45) Date of publication and mention of the grant of the patent: **20.07.2011**
(21) Application number: 08751248.9
(22) Date of filing: 19.05.2008
(51) Int. Cl.: G01N 33/53, C12Q 1/04

(54) **RAPID ASSESSMENT OF UPPER RESPIRATORY CONDITIONS**
SCHNELLE BEURTEILUNG VON LEIDEN DER OBEREN ATEMWEGE
ÉVALUATION RAPIDE D'AFFECTIONS DES VOIES RESPIRATOIRES SUPÉRIEURES

(30) Priority: 30.08.2007 US 847569
(43) Date of publication of application: 16.06.2010
(73) Proprietor: KIMBERLY-CLARK WORLDWIDE, INC., Neenah, WI 54956 (US)
(72) Inventor: MARTIN, Stephanie, M., Woodstock, GA 30188 (US); MACDONALD, J., Gavin, Decatur, GA 30033 (US); LYE, Jason, Atlanta, GA 30316 (US); SAYRE, Curtis, Atlanta, GA 30306 (US); THOMPSON, Kimberlee, Conyers, GA 30094 (US)
(74) Representative: Zimmermann & Partner
(86) International application number: PCT/IB2008/051965
(87) International publication number: WO 2009/027855

(56) References cited:
- WO-A-01/71318
- WO-A-2007/027899
- WO-A-2008/026104
- WO-A-2008/026105
- US-A1- 2004 029 171
- US-A1- 2006 134 613
- US-A1- 2006 210 970
- US-B1- 6 589 761

## Description

### Background of the Invention

Upper respiratory conditions include acute and systemic infections involving the upper respiratory tract (e.g., nose, sinuses, pharynx, larynx, or bronchi), such as rhinosinusitis (common cold), sinusitis, pharyngitis/tonsillitis, laryngitis, bronchitis, influenza (the flu), and so forth. It is common for patients afflicted with respiratory discomfort (e.g., congestion, cough, running nose, sore throat, fever, facial pressure and sneezing) to seek the advice of a clinician in an effort to minimize or overcome their discomfort. However, the clinician presented with such a patient typically has the daunting task of determining which principal etiology is responsible for the discomfort experienced by a particular patient. Common viral etiologies of upper respiratory conditions include, for instance, Rhinoviruses, Coronavirus, Influenza A or B virus, Parainfluenza virus, Adenovirus, etc., while common bacterial etiologies include *Chlamydia pneumoniae*, *Haemophilus influenzae*, *Streptococcus pneumoniae*, *Mycoplasma pneumoniae*, etc. Certain allergies may also lead to upper respiratory conditions. Unfortunately, a misdiagnosis of the proper etiology may be quite problematic. For example, an incorrect diagnosis of an allergy as sinusitis may result in the unnecessary prescription of a course of antibiotics, which would do little to alleviate the allergic discomfort and raise the possibility of a subsequent resistant bacterial infection.

As such, a need currently exists for a technique of rapidly and simply assessing an upper respiratory condition.

US2004/029171 discloses saliva assays for detecting bacteria using various indicators, like pH indicators, redox indicators and fluorescence indicators in various reaction chambers. WO 01/71318 A and US 6 589 761, combine solvatochromatic and pH indicators for detecting bacteria.

### Summary of the Invention

In accordance with one embodiment of the present invention, a method for rapidly detecting microorganisms in an upper respiratory test sample is disclosed. The method comprises contacting a test strip with the upper respiratory test sample. The test strip comprises at least one broad spectrum indicator that exhibits a first spectral response in the presence of bacteria and a second spectral response in the presence of viruses. The test strip further comprises an array that contains at least one differentiating indicator, the array exhibiting a third spectral response in the presence of one type of microorganism and a fourth spectral response in the presence of another type of microorganism. The broad spectrum indicator is observed for the first spectral response or the second spectral response, the presence of the second spectral response indicating the presence of a virus in the sample. Thereafter, the array is observed for the third spectral response or the fourth spectral response.

In accordance with another embodiment of the present invention, a kit for rapidly detecting microorganisms in an upper respiratory test sample is disclosed. The kit comprises a device for collecting a test sample from an upper respiratory tract of a host and a test strip comprising at least one broad spectrum indicator that exhibits a first spectral response in the presence of bacteria and a second spectral response in the presence of viruses. The test strip further comprises an array that contains at least one differentiating indicator, the array exhibiting a third spectral response in the presence of one type of microorganism and a fourth spectral response in the presence of another type of microorganism.

In all embodiments according to the invention the broad spectrum indicator is a solvatochromatic indicator and the differentiating indicator contains a pH-sensitive indicator, metal complexing indicator, or both.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figure in which:

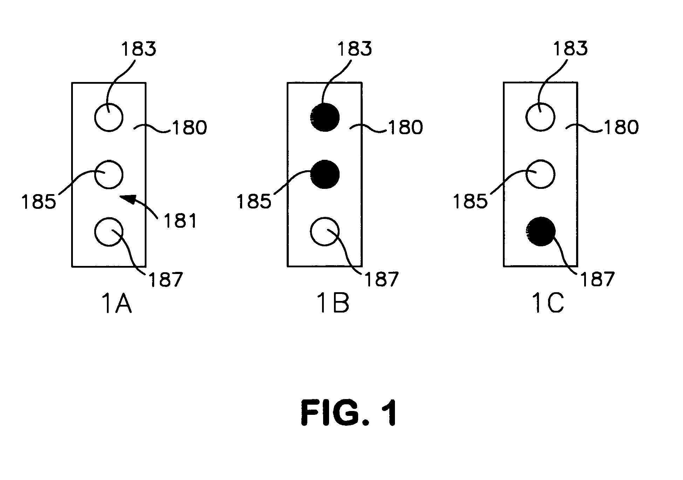

Fig. 1 is a perspective view of an exemplary test strip of the present invention prior to contact with a test sample (Fig. 1A), after contact with a test sample infected with bacteria (Fig. 1B); and after contact with a test sample not infected with bacteria (Fig. 1C).

Repeat use of reference characters in the present specification and drawing is intended to represent same or analogous features or elements of the invention.

### Detailed Description of Representative Embodiments

### Definitions

As used herein, the term "upper respiratory test sample" generally refers to a biological material obtained directly or indirectly from the upper respiratory tract of a host, such as from the nasal passage, mouth, throat, etc. The test sample may be obtained in by any method desired, such as using a swab. The test sample may also be used as obtained or pretreated in some manner. For example, such pretreatment may include filtration, precipitation, dilution, distillation, mixing, concentration, inactivation of interfering components, the addition of reagents, lysing, etc.

As used herein, the term "host" refers to any animal, preferably a human.

### Detailed Description

Reference now will be made in detail to various embodiments of the invention, one or more examples of which are set forth below. Each example is provided by way of explanation of the invention, not limitation of the invention.

The method and kit according to the invention are defined in the claims.

Generally speaking, the present invention is directed to a method for rapidly assessing upper respiratory conditions. More specifically, the method involves contacting a sample obtained from the upper respiratory tract of a host with a test strip. The test strip contains an indicator that provides a broad spectrum response in the presence of bacteria, mold, yeast, or other microorganisms that is different than its response in the presence of viruses. This allows for a rapid and simple assessment as to whether the test sample is infected with a virus or some other microorganism. To help a clinician identify the proper course of treatment, it may also be desirable to obtain further information about the particular type of microorganism present. In this regard, the test strip contains any array of one or more differentiating indicators that provides a certain spectral response in the presence of different types of microorganisms. For example, the array may provide a certain spectral response in the presence of gram-negative bacteria, but a completely different spectral response in the presence of gram-positive bacteria. Likewise, the array may provide a certain spectral response in the presence of Rhinoviruses (associated with the common cold), but a different response in the presence of Influenza viruses. Detection of the spectral response provided by the indicators may thus allow for rapid differentiation between different types of microorganisms.

Any of a variety of microorganisms may be detected in accordance with the present invention. For example, gram-positive (e.g., *Streptococcus pyogenes* and *Streptococcus pneumoniae*) and gram-negative (e.g., *Moraxella lacunata*, *Haemophilus influenzae*, and *Chlamydia pneumoniae*) bacteria are often associated with upper respiratory conditions and may be detected in the present invention. Gram-negative bacteria have a cell wall coated with lipopolysaccharide (LPS). Gram-positive bacteria are coated with thick peptidoglycan (or murein) sheet-like layers. The most prevalent bacterial causes of upper respiratory conditions are *Streptococcus pneumoniae*, *Haemophilus influenzae*, and *Streptococcus pyogenes*. *Streptococcus pyogenes* is a gram-positive, nonmotile, nonsporeforming cocci that occurs in chains or in pairs of cells. *Streptococcus pyogenes* is a catalase-negative aerotolerant anaerobe (facultative anaerobe) and requires enriched medium containing blood in order to grow. *Haemophilus influenzae* is a small, nonmotile Gram-negative bacterium in the family *Pasteurellaceae*. Viruses most commonly associated with upper respiratory conditions are those of the genera Rhinovirus (e.g., Rhinovirus Type 42), Influenzavirus A (e.g., H1N1, H1N2, H2N2 or H3N2 strains), Influenzavirus B, Influenzavirus C, Respiroviruses (e.g., Human Parainfluenza Types 1, 2, 3, and 4), Simplexvirus (e.g., Herpes Simplex Type I and Herpex Simplex Type II), Mastadenovirus (e.g., Adenovirus Types 1, 2, 5, and 6), and Coronavirus (e.g., Human Coronavirus 229E, Human Coronavirus NL63, Human Coronavirus OC43, SARS-CoV, and IBV). Of these common forms of viruses, Simplexviruses and Mastadenoviruses are generally double-stranded DNA viruses that contain icosahedral capsids. Simplexviruses typically possess an enveloped virion, while Mastadenoviruses are naked. Rhinoviruses, Influenza viruses, Parainfluenza viruses, and Coronaviruses are single-stranded RNA viruses. Rhinoviruses contain icosahedral capsids and are not enveloped, Influenza and Parainfluenza viruses contain helical capsids and are enveloped, and Coronaviruses have asymmetrical capsids and are enveloped.

As noted above, an indicator is employed in the present invention that can provide a broad spectrum response for bacteria or other microoranisms that is different than its response for viruses. The present inventors have discovered that solvatochromatic indicators are particularly effective in undergoing a distinct color change in the presence of a broad spectrum of bacteria or other microorganisms, yet very little if any change in the presence of viruses associated with upper respiratory conditions. Merocyanine indicators (e.g., mono-, di-, and tri-merocyanines) are one example of a type of solvatochromatic indicator that may be employed in the present invention. Merocyanine indicators, such as merocyanine 540, fall within the donor - simple acceptor indicator classification of Griffiths as discussed in "Colour and Constitution of Organic Molecules" Academic Press, London (1976). More specifically, merocyanine indicators have a basic nucleus and acidic nucleus separated by a conjugated chain having an even number of methine carbons. Such indicators possess a carbonyl group that acts as an electron acceptor moiety. The electron acceptor is conjugated to an electron donating group, such as a hydroxyl or amino group. The merocyanine indicators may be cyclic or acyclic (e.g., vinylalogous amides of cyclic merocyanine indicators). For example, cyclic merocyanine indicators generally have the following structure: 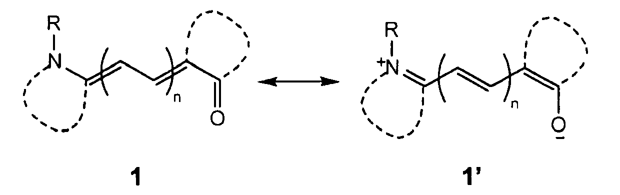 wherein, n is any integer, including 0. As indicated above by the general structures **1** and **1'**, merocyanine indicators typically have a charge separated (i.e., "zwitterionic") resonance form. Zwitterionic indicators are those that contain both positive and negative charges and are net neutral, but highly charged. Without intending to be limited by theory, it is believed that the zwitterionic form contributes significantly to the ground state of the indicator. The color produced by such indicators thus depends on the molecular polarity difference between the ground and excited state of the indicator. One particular example of a merocyanine indicator that has a ground state more polar than the excited state is set forth below as structure **2**.

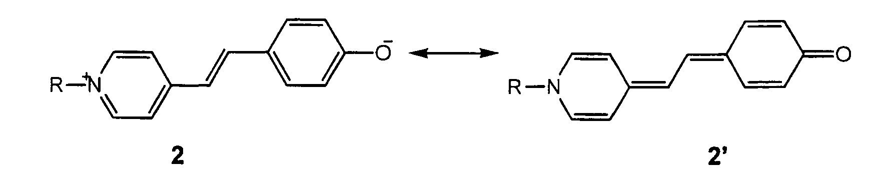

The charge-separated left hand canonical **2** is a major contributor to the ground state whereas the right hand canonical **2'** is a major contributor to the first excited state. Still other examples of suitable merocyanine indicators are set forth below in the following structures **3-13**. 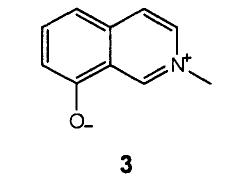 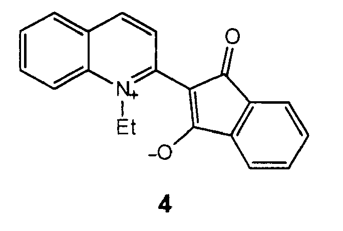 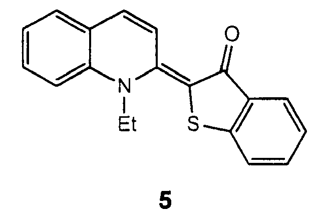 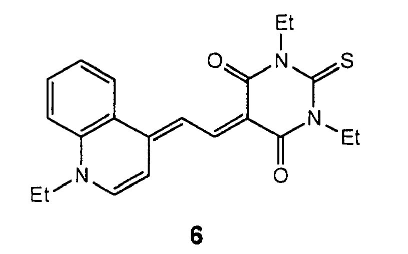 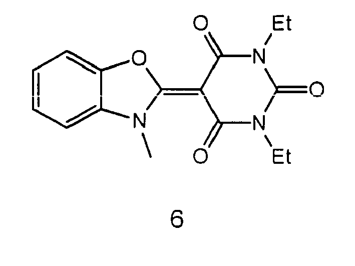 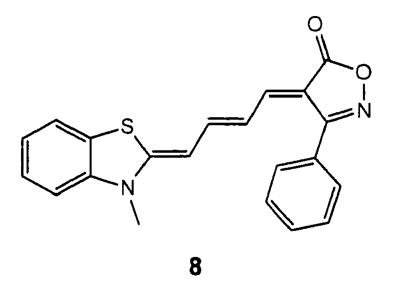 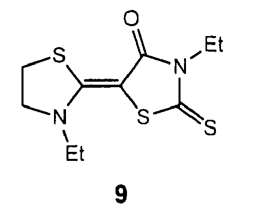 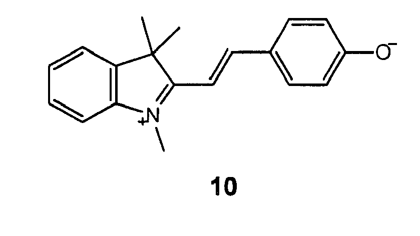 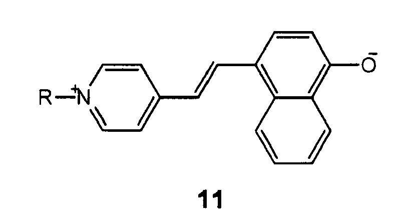 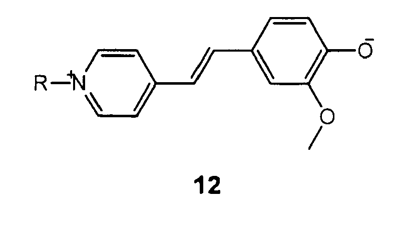 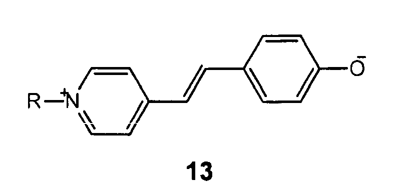 wherein, "R" is a group, such as methyl, alkyl, aryl, phenyl, etc.

Indigo is another example of a suitable solvatochromatic indicator for use in the present invention. Indigo has a ground state that is significantly less polar than the excited state. For example, indigo generally has the following structure **14**:

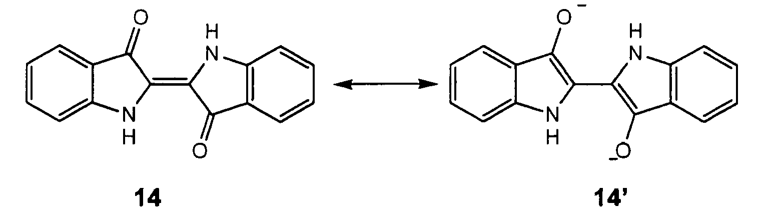

The left hand canonical form **14** is a major contributor to the ground state of the indicator, whereas the right hand canonical **14'** is a major contributor to the excited state.

Other suitable solvatochromatic indicators that may be used in the present invention include those that possess a permanent zwitterionic form. That is, these indicators have formal positive and negative charges contained within a contiguous π- electron system. Contrary to the merocyanine indicators referenced above, a neutral resonance structure cannot be drawn for such permanent zwitterionic indicators. Exemplary indicators of this class include *N*-phenolate betaine indicators, such as those having the following general structure: 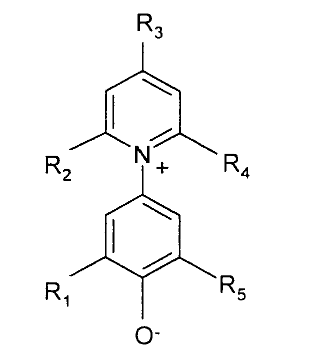 wherein R₁-R₅ are independently selected from the group consisting of hydrogen, a nitro group (e.g., nitrogen), a halogen, or a linear, branched, or cyclic C₁ to C₂₀ group (e.g., alkyl, phenyl, aryl, pyridinyl, etc.), which may be saturated or unsaturated and unsubstituted or optionally substituted at the same or at different carbon atoms with one, two or more halogen, nitro, cyano, hydroxy, alkoxy, amino, phenyl, aryl, pyridinyl, or alkylamino groups. For example, the N-phenolate betaine indicator may be 4-(2,4,6-triphenylpyridinium-1-yl)-2,6-diphenylphenolate (Reichardt's dye) having the following general structure **15**:

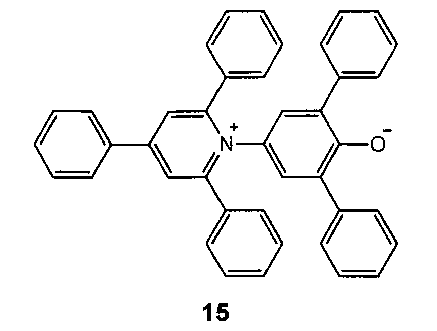

Reichardt's dye shows strong negative solvatochromism and may thus undergo a significant color change from blue to colorless in the presence of bacteria. That is, Reichardt's dye displays a shift in absorbance to a shorter wavelength and thus has visible color changes as solvent eluent strength (polarity) increases. Still other examples of suitable negatively solvatochromatic pyridinium N-phenolate betaine indicators are set forth below in structures **16-23**: 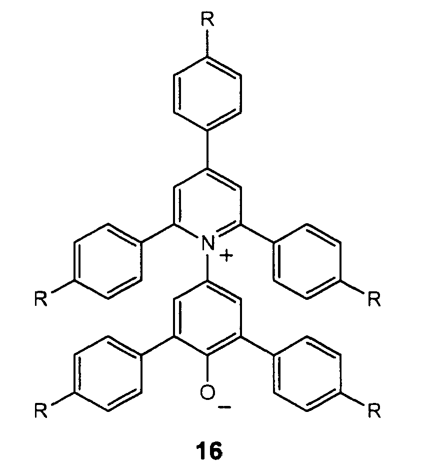 wherein, R is hydrogen, -C(CH₃)₃, -CF₃, or C₆F₁₃.

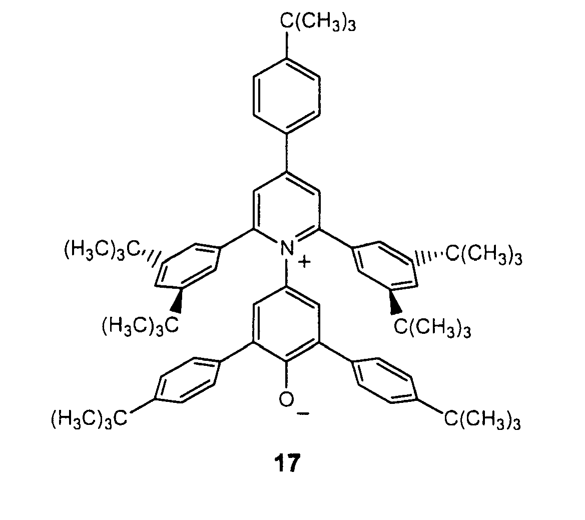

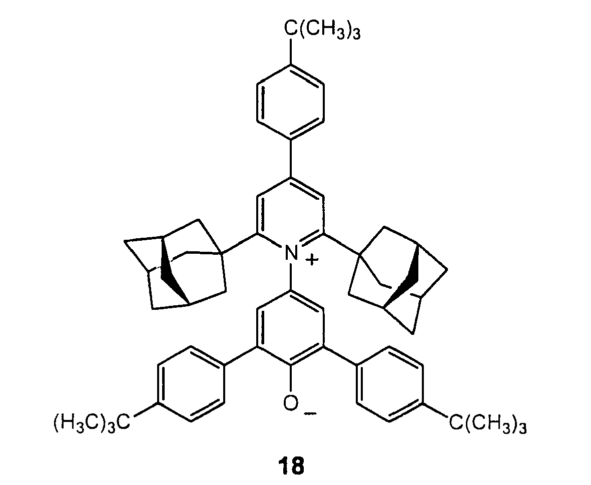

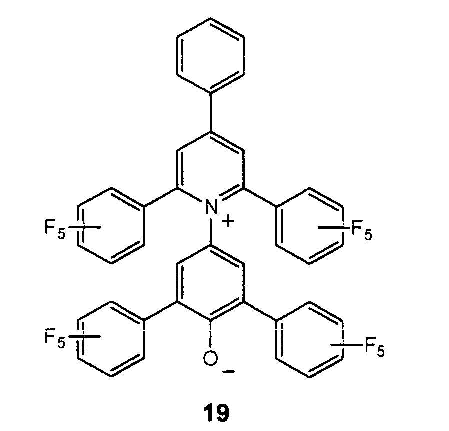

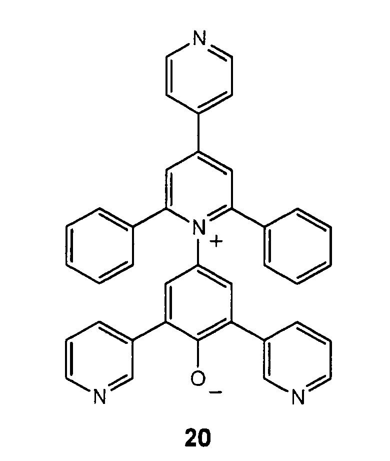

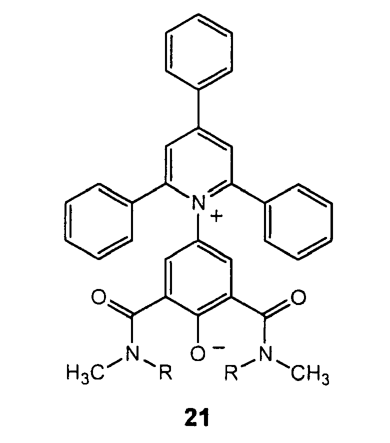

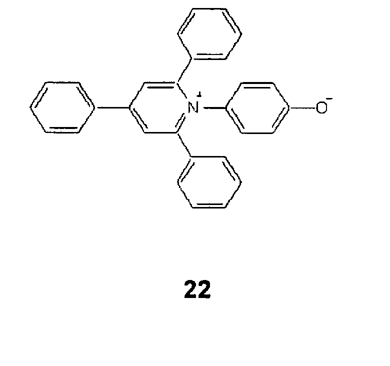

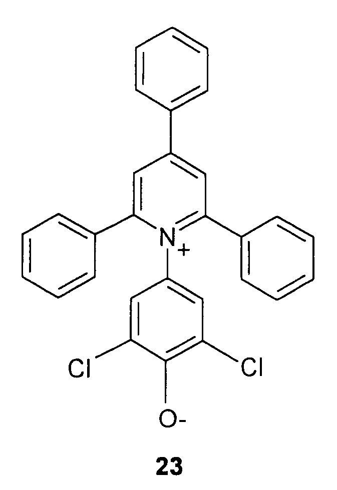

Still additional examples of indicators having a permanent zwitterionic form include indicators having the following general structure **24**: 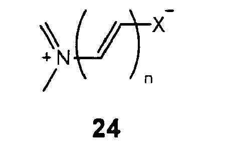 wherein, n is 0 or greater, and X is oxygen, carbon, nitrogen, sulfur, etc. Particular examples of the permanent zwitterionic indicator shown in structure **24** include the following structures **25 - 33**.

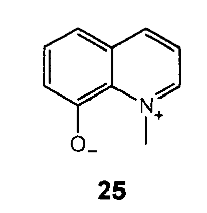

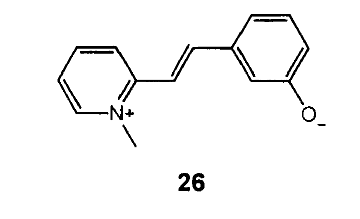

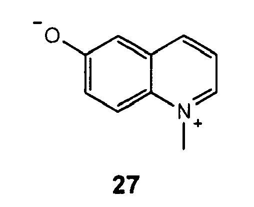

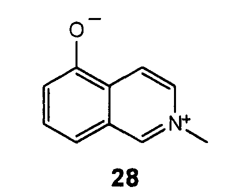

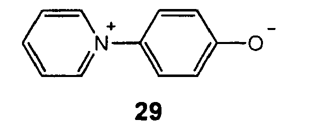

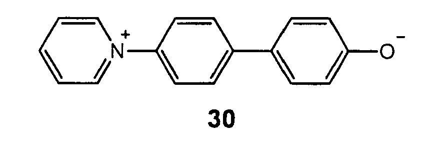

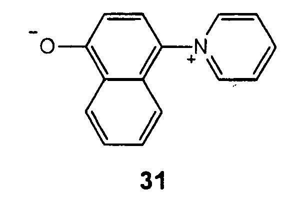

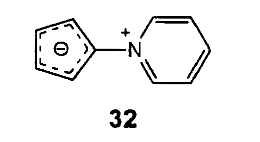

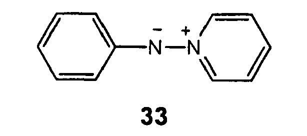

Still other suitable solvatochromatic indicators may include, but are not limited to 4-dicyanmethylene-2-methyl-6-(p-dimethylaminostyryl)-4H-pyran (DCM); 6-propionyl-2-(dimethylamino)naphthalene (PRODAN); 9-(diethylamino)-5H-benzo[a]phenox-azin-5-one (Nile Red); 4-(dicyanovinyl)julolidine (DCVJ); phenol blue; stilbazolium indicators; coumarin indicators; ketocyanine indicators; N,N-dimethyl-4-nitroaniline (NDMNA) and N-methyl-2-nitroaniline (NM2NA); Nile blue; 1-anilinonaphthalene-8-sulfonic acid (1,8-ANS), and dapoxylbutylsulfonamide (DBS) and other dapoxyl analogs. Besides the above-mentioned indicators, still other suitable indicators that may be used in the present invention include, but are not limited to, 4-[2-N-substituted-1,4-hydropyridin-4-ylidine)ethylidene]cyclohexa-2,5-dien-1-one, red pyrazolone indicators, azomethine indicators, indoaniline indicators, and mixtures thereof.

In addition to a broad spectrum indicator, one or more indicators (e.g., dyes, pigments, etc.) are also employed that are capable of differentiating between certain types of microorganisms. pH-sensitive indicators, for instance, may be employed that can detect a change in the pH of the growth medium of the microorganism. Bacteria and viruses, for instance, may metabolize the growth medium and generate acidic compounds (e.g., CO₂) or basic compounds (e.g., ammonia) that lead to a change in pH. Likewise, certain microorganisms (e.g., bacteria) contain highly organized acid moieties on their cell walls. Because the acidic/basic shift may vary for different microorganisms, pH-sensitive indicators may be selected in the present invention that are tuned for the desired pH transition. In this manner, the test strip may be provided with pH-sensitive indicators that are configured to undergo a detectable color change only in the presence of bacteria or viruses exhibiting a certain acidic/basic shift.

Phthalein indicators constitute one class of suitable pH-sensitive indicators that may be employed in the test strip of the present invention. Phenol Red (i.e., phenolsulfonephthalein), for example, exhibits a transition from yellow to red over the pH range 6.6 to 8.0. Above a pH of about 8.1, Phenol Red turns a bright pink (fuschia) color. Derivatives of Phenol Red may also be suitable for use in the present invention, such as those substituted with chloro, bromo, methyl, sodium carboxylate, carboxylic acid, hydroxyl and amine functional groups. Exemplary substituted Phenol Red compounds include, for instance, Chlorophenol Red, Metacresol Purple (meta-cresolsulfonephthalein), Cresol Red (ortho-cresolsulfonephthalein), Pyrocatecol Violet (pyrocatecolsulfonephthalein), Chlorophenol Red (3',3"-dichlorophenolsulfonephthalein), Xylenol Blue (the sodium salt of para-xylenolsulfonephthalein), Xylenol Orange, Mordant Blue 3 (C.I. 43820), 3,4,5,6-tetrabromophenolsulfonephthalein, Bromoxylenol Blue, Bromophenol Blue (3',3",5',5"-tetrabromophenolsulfonephthalein), Bromochlorophenol Blue (the sodium salt of dibromo-5',5"-dichlorophenolsulfonephthalein), Bromocresol Purple (5',5"-dibromo-ortho-cresolsulfonephthalein), Bromocresol Green (3',3",5',5"-tetrabromo-ortho-cresolsulfonephthalein), and so forth. Still other suitable phthalein indicators are well known in the art, and may include Bromothymol Blue, Thymol Blue, Bromocresol Purple, thymolphthalein, and phenolphthalein (a common component of universal indicators). For example, Chlorophenol Red exhibits a transition from yellow to red over a pH range of about 4.8 to 6.4; Bromothymol Blue exhibits a transition from yellow to blue over a pH range of about 6.0 to 7.6; thymolphthalein exhibits a transition from colorless to blue over a pH range of about 9.4 to 10.6; phenolphthalein exhibits a transition from colorless to pink over a pH range of about 8.2 to 10.0; Thymol Blue exhibits a first transition from red to yellow over a pH range of about 1.2 to 2.8 and a second transition from yellow to pH over a pH range of 8.0 to 9.6; Bromophenol Blue exhibits a transition from yellow to violet over a pH range of about 3.0 to 4.6; Bromocresol Green exhibits a transition from yellow to blue over a pH range of about 3.8 to 5.4; and Bromocresol Purple exhibits a transition from yellow to violet over a pH of about 5.2 to 6.8.

Hydroxyanthraquinones constitute another suitable class of pH-sensitive indicators for use in the present invention. Hydroxyanthraquinones have the following general structure:

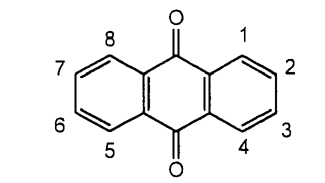

The numbers 1-8 shown in the general formula represent a location on the fused ring structure at which substitution of a functional group may occur. For hydroxyanthraquinones, at least one of the functional groups is or contains a hydroxy (-OH) group. Other examples of functional groups that may be substituted on the fused ring structure include halogen groups (e.g., chlorine or bromine groups), sulfonyl groups (e.g., sulfonic acid salts), alkyl groups, benzyl groups, amino groups (e.g., primary, secondary, tertiary, or quaternary amines), carboxy groups, cyano groups, phosphorous groups, etc. Some suitable hydroxyanthraquinones that may be used in the present invention, Mordant Red 11 (Alizarin), Mordant Red 3 (Alizarin Red S), Alizarin Yellow R, Alizarin Complexone, Mordant Black 13 (Alizarin Blue Black B), Mordant Violet 5 (Alizarin Violet 3R), Alizarin Yellow GG, Natural Red 4 (carminic acid), amino-4-hydroxyanthraquinone, Emodin, Nuclear Fast Red, Natural Red 16 (Purpurin), Quinalizarin, and so forth. For instance, carminic acid exhibits a first transition from orange to red over a pH range of about 3.0 to 5.5 and a second transition from red to purple over a pH range of about 5.5 to 7.0. Alizarin Yellow R, on the other hand, exhibits a transition from yellow to orange-red over a pH range of about 10.1 to 12.0.

Yet another suitable class of pH-sensitive indicators that may be employed in the test strip is aromatic azo compounds having the general structure:

X-R₁-N=N-R₂-Y

wherein,
R₁ is an aromatic group;
R₂ is selected from the group consisting of aliphatic and aromatic groups; and
X and Y are independently selected from the group consisting of hydrogen, halides, -NO₂, -NH₂, aryl groups, alkyl groups, alkoxy groups, sulfonate groups, - SO₃H, -OH, -COH, -COOH, halides, etc. Also suitable are azo derivatives, such as azoxy compounds (X-R₁-N=NO-R₂-Y) or hydrazo compounds (X-R₁-NH-NH-R₂-Y). Particular examples of such azo compounds (or derivatives thereof) include Methyl Violet, Methyl Yellow, Methyl Orange, Methyl Red, and Methyl Green. For instance, Methyl Violet undergoes a transition from yellow to blue-violet at a pH range of about 0 to 1.6, Methyl Yellow undergoes a transition from red to yellow at a pH range of about 2.9 to 4.0, Methyl Orange undergoes a transition from red to yellow at a pH range of about 3.1 to 4.4, and Methyl Red undergoes a transition from red to yellow at a pH range of about 4.2 to 6.3.

Arylmethanes (e.g., diarylmethanes and triarylmethanes) constitute still another class of suitable pH-sensitive indicators for use in the present invention. Triarylmethane leuco bases, for example, have the following general structure: 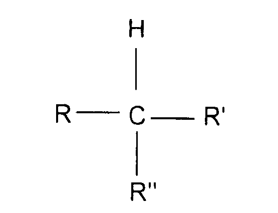 wherein R, R', and R" are independently selected from substituted and unsubstituted aryl groups, such as phenyl, naphthyl, anthracenyl, etc. The aryl groups may be substituted with functional groups, such as amino, hydroxyl, carbonyl, carboxyl, sulfonic, alkyl, and/or other known functional groups. Examples of such triarylmethane leuco bases include Leucomalachite Green, Pararosaniline Base, Crystal Violet Lactone, Crystal Violet Leuco, Crystal Violet, Cl Basic Violet 1, Cl Basic Violet 2, Cl Basic Blue, Cl Victoria Blue, N-benzoyl leuco-methylene, etc. Likewise suitable diarylmethane leuco bases may include 4,4'-bis (dimethylamino) benzhydrol (also known as "Michler's hydrol"), Michler's hydrol leucobenzotriazole, Michler's hydrol leucomorpholine, Michler's hydrol leucobenzenesulfonamide, etc. In one particular embodiment, the indicator is Leucomalachite Green Carbinol (Solvent Green 1) or an analog thereof, which is normally colorless and has the following structure:

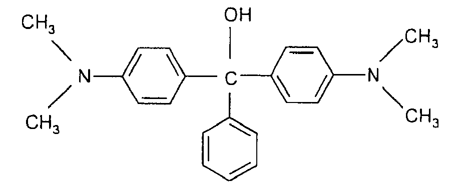

Under acidic conditions, one or more free amino groups of the Leucomalachite Green Carbinol form may be protonated to form Malachite Green (also known as Aniline Green, Basic Green 4, Diamond Green B, or Victoria Green B), which has the following structure:

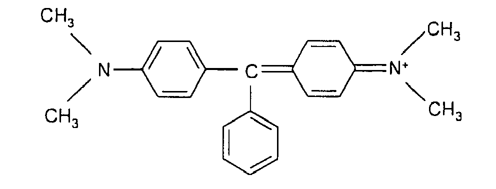

Malachite Green typically exhibits a transition from yellow to blue-green over a pH range 0.2 to 1.8. Above a pH of about 1.8, malachite green turns a deep green color.

Still other suitable pH-sensitive indicators that may be employed in the test strip include Congo Red, Litmus (azolitmin), Methylene Blue, Neutral Red, Acid Fuchsin, Indigo Carmine, Brilliant Green, Picric acid, Metanil Yellow, m-Cresol Purple, Quinaldine Red, Tropaeolin OO, 2,6-dinitrophenol, Phloxine B, 2,4-dinitrophenol, 4-dimethylaminoazobenzene, 2,5-dinitrophenol, 1-Naphthyl Red, Chlorophenol Red, Hematoxylin, 4-nitrophenol, nitrazine yellow, 3-nitrophenol, Alkali Blue, Epsilon Blue, Nile Blue A, universal indicators, and so forth. For instance, Congo Red undergoes a transition from blue to red at a pH range of about 3.0 to 5.2, Litmus undergoes a transition from red to blue at a pH range of about 4.5 to 8.3, and Neutral Red undergoes a transition from red to yellow at a pH range of about 11.4 to 13.0.

In addition to pH, other mechanisms may also be wholly or partially responsible for inducing a color change in the indicators. For example, many microorganisms (e.g., bacteria) produce low molecular weight iron-complexing compounds in growth media, which are known as "siderophores." Metal complexing indicators may thus be employed in some embodiments of the present invention that undergo a color change in the presence of siderophores. One particularly suitable class of metal complexing indicators are aromatic azo compounds, such as Eriochrome Black T, Eriochrome Blue SE (Plasmocorinth B), Eriochrome Blue Black B, Eriochrome Cyanine R, Xylenol Orange, Chrome Azurol S, carminic acid, etc. Still other suitable metal complexing indicators may include Alizarin Complexone, Alizarin S, Arsenazo III, Aurintricarboxylic acid, 2,2'-Bipyidine, Bromopyrogallol Red, Calcon (Eriochrome Blue Black R), Calconcarboxylic acid, Chromotropic acid, disodium salt, Cuprizone, 5-(4-Dimethylamino-benzylidene)rhodanine, Dimethylglyoxime, 1,5-Diphenylcarbazide, Dithizone, Fluorescein Complexone, Hematoxylin, 8-Hydroxyquinoline, 2-Mercaptobenzothiazole, Methylthymol Blue, Murexide, 1-Nitroso-2-naphthol, 2-Nitroso-1-naphthol, Nitroso-R-salt, 1,10-Phenanthroline, Phenylfluorone, Phthalein Purple, 1-(2-Pyridylazo)-naphthol, 4-(2-Pyridylazo)resorcinol, Pyrogallol Red, Sulfonazo III. 5-Sulfosalicylic acid, 4-(2-Thiazolylazo)resorcinol, Thorin, Thymolthalexon, Tiron, Tolurnr-3,4-dithiol, Zincon, and so forth. It should be noted that one or more of the pH-sensitive indicators referenced above may also be classified as metal complexing indicators.

Although the above-referenced indicators are classified based on their mechanism of color change (e.g., pH-sensitive, metal complexing, or solvatochromatic), it should be understood that the present invention is not limited to any particular mechanism for the color change. Even when a pH-sensitive indicator is employed, for instance, other mechanisms may actually be wholly or partially responsible for the color change of the indicator. For example, redox reactions between the indicator and microorganism may contribute to the color change.

To form the test strip of the present invention, the indicators may be applied to a substrate, such as a film, paper, nonwoven web, knitted fabric, woven fabric, foam, glass, etc. For example, the materials used to form the substrate may include, but are not limited to, natural, synthetic, or naturally occurring materials that are synthetically modified, such as polysaccharides (e.g., cellulose materials such as paper and cellulose derivatives, such as cellulose acetate and nitrocellulose); polyether sulfone; polyethylene; nylon; polyvinylidene fluoride (PVDF); polyester; polypropylene; silica; inorganic materials, such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon or rayon); porous gels, such as silica gel, agarose, dextran, and gelatin; polymeric films, such as polyacrylamide; and so forth.

If desired, an indicator may be applied in the form of a composition that contains a mobile carrier. The carrier may be a liquid, gas, gel, etc., and may be selected to provide the desired performance (time for change of color, contrast between different areas, and sensitivity) of the indicator. In some embodiments, for instance, the carrier may be an aqueous solvent, such as water, as well as a non-aqueous solvent, such as glycols (e.g., propylene glycol, butylene glycol, triethylene glycol, hexylene glycol, polyethylene glycols, ethoxydiglycol, and dipropyleneglycol); alcohols (e.g., methanol, ethanol, n-propanol, and isopropanol); triglycerides; ethyl acetate; acetone; triacetin; acetonitrile, tetrahydrafuran; xylenes; formaldehydes (e.g., dimethylformamide, "DMF"); etc.

Other additives may also be applied to the test strip, either separately or in conjunction with an indicator composition. In one embodiment, for instance, cyclodextrins are employed that are believed to inhibit the crystallization of the indicator and thus provide a more vivid color and also enhance detection sensitivity. That is, single indicator molecules have greater sensitivity for microorganisms because each indicator molecule is free to interact with the microbial membrane. In contrast, small crystals of indicator have to first dissolve and then penetrate the membrane. Examples of suitable cyclodextrins may include, but are not limited to, hydroxypropyl-β-cyclodextrin, hydroxyethyl-β-cyclodextrin, γ-cyclodextrin, hydroxypropyl-γ-cyclodextrin, and hydroxyethyl-γ-cyclodextrin, which are commercially available from Cerestar International of Hammond, Indiana.

Surfactants may also help enhance the contrast between different indicators. Particularly desired surfactants are nonionic surfactants, such as ethoxylated alkylphenols, ethoxylated and propoxylated fatty alcohols, ethylene oxide-propylene oxide block copolymers, ethoxylated esters of fatty (C₈-C₁₈) acids, condensation products of ethylene oxide with long chain amines or amides, condensation products of ethylene oxide with alcohols, acetylenic diols, and mixtures thereof. Various specific examples of suitable nonionic surfactants include, but are not limited to, methyl gluceth-1 0, PEG-20 methyl glucose distearate, PEG-20 methyl glucose sesquistearate, C₁₁₋₁₅ pareth-20, ceteth-8, ceteth-12, dodoxynol-12, laureth-15, PEG-20 castor oil, polysorbate 20, steareth-20, polyoxyethylene-10 cetyl ether, polyoxyethylene-10 stearyl ether, polyoxyethylene-20 cetyl ether, polyoxyethylene-10 oleyl ether, polyoxyethylene-20 oleyl ether, an ethoxylated nonylphenol, ethoxylated octylphenol, ethoxylated dodecylphenol, or ethoxylated fatty (C₆-C₂₂) alcohol, including 3 to 20 ethylene oxide moieties, polyoxyethylene-20 isohexadecyl ether, polyoxyethylene-23 glycerol laurate, polyoxy-ethylene-20 glyceryl stearate, PPG-10 methyl glucose ether, PPG-20 methyl glucose ether, polyoxyethylene-20 sorbitan monoesters, polyoxyethylene-80 castor oil, polyoxyethylene-15 tridecyl ether, polyoxy-ethylene-6 tridecyl ether, laureth-2, laureth-3, laureth-4, PEG-3 castor oil, PEG 600 dioleate, PEG 400 dioleate, and mixtures thereof. Commercially available nonionic surfactants may include the SURFYNOL® range of acetylenic diol surfactants available from Air Products and Chemicals of Allentown, Pennsylvania and the TWEEN® range of polyoxyethylene surfactants available from Fischer Scientific of Pittsburgh, Pennsylvania.

A binder may also be employed to facilitate the immobilization of an indicator on the substrate. For example, water-soluble organic polymers may be employed as binders, such as polysaccharides and derivatives thereof. Polysaccharides are polymers containing repeated carbohydrate units, which may be cationic, anionic, nonionic, and/or amphoteric. In one particular embodiment, the polysaccharide is a nonionic, cationic, anionic, and/or amphoteric cellulosic ether. Suitable nonionic cellulosic ethers may include, but are not limited to, alkyl cellulose ethers, such as methyl cellulose and ethyl cellulose; hydroxyalkyl cellulose ethers, such as hydroxyethyl cellulose, hydroxypropyl cellulose, hydroxypropyl hydroxybutyl cellulose, hydroxyethyl hydroxypropyl cellulose, hydroxyethyl hydroxybutyl cellulose and hydroxyethyl hydroxypropyl hydroxybutyl cellulose; alkyl hydroxyalkyl cellulose ethers, such as methyl hydroxyethyl cellulose, methyl hydroxypropyl cellulose, ethyl hydroxyethyl cellulose, ethyl hydroxypropyl cellulose, methyl ethyl hydroxyethyl cellulose and methyl ethyl hydroxypropyl cellulose; and so forth.

Suitable techniques for applying an indicator composition to a substrate include printing, dipping, spraying, melt extruding, coating (e.g., solvent coating, powder coating, brush coating, etc.), spraying, and so forth. Printing techniques may include, for instance, gravure printing, flexographic printing, screen printing, laser printing, thermal ribbon printing, piston printing, etc. In one particular embodiment, ink-jet printing techniques are employed to apply an indicator to the substrate. Ink-jet printing is a non-contact printing technique that involves forcing an ink through a tiny nozzle (or a series of nozzles) to form droplets that are directed toward the substrate. Two techniques are generally utilized, i.e., "DOD" (Drop-On-Demand) or "continuous" ink-jet printing. In continuous systems, ink is emitted in a continuous stream under pressure through at least one orifice or nozzle. The stream is perturbed by a pressurization actuator to break the stream into droplets at a fixed distance from the orifice. DOD systems, on the other hand, use a pressurization actuator at each orifice to break the ink into droplets. The pressurization actuator in each system may be a piezoelectric crystal, an acoustic array, a thermal array, etc. The selection of the type of ink jet system varies on the type of material to be printed from the print head. For example, conductive materials are sometimes required for continuous systems because the droplets are deflected electrostatically. Thus, when the sample channel is formed from a dielectric material, DOD printing techniques may be more desirable.

An indicator composition may be formed as a printing ink using any of a variety of known components and/or methods. For example, the printing ink may contain water as a carrier, and particularly deionized water. Various co-carriers may also be included in the ink, such as lactam, N-methyl pyrrolidone, N-methylacetamide, N-methylmorpholine-N-oxide, N,N-dimethylacetamide, N-methyl formamide, propyleneglycol-monomethylether, tetramethylene sulfone, tripropyleneglycolmonomethylether, propylene glycol, and triethanolamine (TEA). Humectants may also be utilized, such as ethylene glycol; diethylene glycol; glycerine; polyethylene glycol 200, 300, 400, and 600; propane 1,3 diol; propylene-glycolmonomethyl ethers, such as Dowanol PM (Gallade Chemical Inc., Santa Ana, CA); polyhydric alcohols; or combinations thereof. Other additives may also be included to improve ink performance, such as a chelating agent to sequester metal ions that could become involved in chemical reactions over time, a corrosion inhibitor to help protect metal components of the printer or ink delivery system, and a surfactant to adjust the ink surface tension. Various other components for use in an ink, such as colorant stabilizers, photoinitiators, binders, surfactants, electrolytic salts, pH adjusters, etc., may be employed as described in U.S. Patent Nos. 5,681,380 to Nohr, et al. and 6,542,379 to Nohr, et al., which are incorporated herein in their entirety by reference thereto for all purposes.

The exact quantity of an indicator employed may vary based on a variety of factors, including the sensitivity of the indicator, the presence of other additives, the desired degree of detectability (e.g., with an unaided eye), the suspected concentration of the microorganism, etc. In some cases, it is desirable to only detect the presence of microorganisms at concentrations that are certain threshold concentrations (e.g., pathogenic). For example, a bacterial concentration of about 1 x 10³ colony forming units ("CFU") per milliliter of a test sample or more, in some embodiments about 1 x 10⁵ CFU/ml or more, in some embodiments about 1 x 10⁶ CFU/ml or more, and in some embodiments, about 1 x 10⁷ CFU/ml may be detected in the present invention. Thus, indicators may be employed in an amount sufficient to undergo a detectable color change in the presence of bacteria at a concentration of at least about 1 x 10³ CFU per milliliter of the test sample. For instance, the indicator may be applied at a concentration from about 0.1 to about 100 milligrams per milliliter of carrier, in some embodiments from about 0.5 to about 60 milligrams per milliliter of carrier, and in some embodiments, from about 1 to about 40 milligrams per milliliter of carrier.

The degree to which an indicator changes color may be determined either visually or using instrumentation. In one embodiment, color intensity is measured with an optical reader. The actual configuration and structure of the optical reader may generally vary as is readily understood by those skilled in the art. Typically, the optical reader contains an illumination source that is capable of emitting electromagnetic radiation and a detector that is capable of registering a signal (e.g., transmitted or reflected light). The illumination source may be any device known in the art that is capable of providing electromagnetic radiation, such as light in the visible or near-visible range (e.g., infrared or ultraviolet light). For example, suitable illumination sources that may be used in the present invention include, but are not limited to, light emitting diodes (LED), flashlamps, cold-cathode fluorescent lamps, electroluminescent lamps, and so forth. The illumination may be multiplexed and/or collimated. In some cases, the illumination may be pulsed to reduce any background interference. Further, illumination may be continuous or may combine continuous wave (CW) and pulsed illumination where multiple illumination beams are multiplexed (e.g., a pulsed beam is multiplexed with a CW beam), permitting signal discrimination between a signal induced by the CW source and a signal induced by the pulsed source. For example, in some embodiments, LEDs (e.g., aluminum gallium arsenide red diodes, gallium phosphide green diodes, gallium arsenide phosphide green diodes, or indium gallium nitride violet/blue/ultraviolet (UV) diodes) are used as the pulsed illumination source. One commercially available example of a suitable UV LED excitation diode suitable for use in the present invention is Model NSHU55OE (Nichia Corporation), which emits 750 to 1000 microwatts of optical power at a forward current of 10 milliamps (3.5-3.9 volts) into a beam with a full-width at half maximum of 10 degrees, a peak wavelength of 370-375 nanometers, and a spectral half-width of 12 nanometers.

In some cases, the illumination source may provide diffuse illumination to the indicator. For example, an array of multiple point light sources (e.g., LEDs) may simply be employed to provide relatively diffuse illumination. Another particularly desired illumination source that is capable of providing diffuse illumination in a relatively inexpensive manner is an electroluminescent (EL) device. An EL device is generally a capacitor structure that utilizes a luminescent material (e.g., phosphor particles) sandwiched between electrodes, at least one of which is transparent to allow light to escape. Application of a voltage across the electrodes generates a changing electric field within the luminescent material that causes it to emit light.

The detector may generally be any device known in the art that is capable of sensing a signal. For instance, the detector may be an electronic imaging detector that is configured for spatial discrimination. Some examples of such electronic imaging sensors include high speed, linear charge-coupled devices (CCD), charge-injection devices (CID), complementary-metal-oxide-semiconductor (CMOS) devices, and so forth. Such image detectors, for instance, are generally two-dimensional arrays of electronic light sensors, although linear imaging detectors (e.g., linear CCD detectors) that include a single line of detector pixels or light sensors, such as, for example, those used for scanning images, may also be used. Each array includes a set of known, unique positions that may be referred to as "addresses." Each address in an image detector is occupied by a sensor that covers an area (e.g., an area typically shaped as a box or a rectangle). This area is generally referred to as a "pixel" or pixel area. A detector pixel, for instance, may be a CCD, CID, or a CMOS sensor, or any other device or sensor that detects or measures light. The size of detector pixels may vary widely, and may in some cases have a diameter or length as low as 0.2 micrometers.

In other embodiments, the detector may be a light sensor that lacks spatial discrimination capabilities. For instance, examples of such light sensors may include photomultiplier devices, photodiodes, such as avalanche photodiodes or silicon photodiodes, and so forth. Silicon photodiodes are sometimes advantageous in that they are inexpensive, sensitive, capable of high-speed operation (short risetime / high bandwidth), and easily integrated into most other semiconductor technology and monolithic circuitry. In addition, silicon photodiodes are physically small, which enables them to be readily incorporated into various types of detection systems. If silicon photodiodes are used, then the wavelength range of the emitted signal may be within their range of sensitivity, which is 400 to 1100 nanometers.

Optical readers may generally employ any known detection technique, including, for instance, luminescence (e.g., fluorescence, phosphorescence, etc.), absorbance (e.g., fluorescent or non-fluorescent), diffraction, etc. In one particular embodiment of the present, the optical reader measures color intensity as a function of absorbance. In one embodiment, absorbance readings are measured using a microplate reader from Dynex Technologies of Chantilly, Virginia (Model # MRX). In another embodiment, absorbance readings are measured using a conventional test known as "CIELAB", which is discussed in Pocket Guide to Digital Printing by F. Cost, Delmar Publishers, Albany, NY. ISBN 0-8273-7592-1 at pages 144 and 145. This method defines three variables, L*, a*, and b*, which correspond to three characteristics of a perceived color based on the opponent theory of color perception. The three variables have the following meaning:
L* = Lightness (or luminosity), ranging from 0 to 100, where 0 = dark and 100= light;
a* = Red/green axis, ranging approximately from -100 to 100; positive values are reddish and negative values are greenish; and
b* = Yellow/blue axis, ranging approximately from -100 to 100; positive values are yellowish and negative values are bluish.

Because CIELAB color space is somewhat visually uniform, a single number may be calculated that represents the difference between two colors as perceived by a human. This difference is termed ΔE and calculated by taking the square root of the sum of the squares of the three differences (ΔL*, Δa*, and Δb*) between the two colors. In CIELAB color space, each ΔE unit is approximately equal to a "just noticeable" difference between two colors. CIELAB is therefore a good measure for an objective device-independent color specification system that may be used as a reference color space for the purpose of color management and expression of changes in color. Using this test, color intensities (L*, a*, and b*) may thus be measured using, for instance, a handheld spectrophotometer from Minolta Co. Ltd. of Osaka, Japan (Model # CM2600d). This instrument utilizes the D/8 geometry conforming to CIE No. 15, ISO 7724/1, ASTME1164 and JIS Z8722-1982 (diffused illumination/ 8-degree viewing system. The D65 light reflected by the specimen surface at an angle of 8 degrees to the normal of the surface is received by the specimen-measuring optical system. Still another suitable optical reader is the reflectance spectrophotometer described in U.S. Patent App. Pub. No. 2003/0119202 to Kaylor, et al., which is incorporated herein in its entirety by reference thereto for all purposes. Likewise, transmission-mode detection systems may also be used in the present invention.

The above-described screening techniques may be implemented in a variety of ways in accordance with the present invention. For example, a test strip may be utilized that contains a detection zone that provides any number of distinct detection regions (e.g., lines, dots, stripes, etc.) so that a user may better determine the presence of viruses, bacteria, or other microorganisms within a test sample. Each region may contain the same indicator, or may contain different indicators for reacting with different types of microorganisms. In one particular embodiment, the test strip contains an array of indicators that provides a distinct spectral response (e.g., pattern of colors) or "fingerprint" for certain types of viruses. For instance, the array may provide a certain spectral response in the presence of Rhinoviruses, but a completely different response in the presence of Influenza viruses, Human Parainfluenza viruses, or other viruses commonly associated with upper respiratory conditions. Similarly, the array may provide a certain spectral response in the presence of gram-positive bacteria, but a completely different response in the presence of gram-negative bacteria.

When employed, the array may contain a plurality of discrete regions (referred to as "addresses") spaced apart in a predetermined pattern. The addresses contain an indicator capable of exhibiting a color change in the presence of a particular microorganism. The selection of indicators for the array is not critical to the present invention so long as the array produces a distinct spectral response. The individual array addresses may be configured in a variety of ways to accomplish this purpose. In one particular embodiment, individual array addresses may contain indicators that each exhibits a distinct spectral response in the presence of specific types of viruses, bacteria, or other microorganisms. Of course, the spectral distinction between individual array addresses need not always be provided by the use of different indicators. For example, the same indicators may be used in individual array addresses, but at a different concentration so as to produce a different spectral response. Certain addresses may likewise contain the same indicator at the same concentration, so long as the array as whole is capable of producing a distinct spectral response.

Apart from the composition of the individual array addresses, a variety of other aspects of the array may be selectively controlled to enhance its ability to provide a distinct spectral response. One factor that influences the ability of the array to produce a distinct spectral response is the number of array addresses employed. Namely, a greater number of individual array addresses may enhance the degree that the spectral response varies for different microorganisms. However, an overly large number of addresses can also lead to difficulty in visually differentiating between spectral responses. Thus, in most embodiments of the present invention, the array contains from 2 to 50 array addresses, in some embodiments from 3 to about 40 array addresses, and in some embodiments, from 4 to 20 array addresses. The number of addresses employed in the array will ultimately depend, at least in part, on the nature of the selected indicators. That is, if the selected indicators have a similar color change in the presence of a microorganism, a larger number of addresses may be needed to provide the desired spectral response.

Another aspect of the array that may influence its ability to provide a distinctive spectral response is the pattern (e.g., size, spacing, alignment, etc.) of the individual array addresses. The individual array addresses may possess a size effective to permit visual observation without unduly increasing the size of the test strip. The size of the addresses may, for example, range from about 0.01 to about 100 millimeters, in some embodiments from about 0.1 to about 50 millimeters, and in some embodiments, from about 1 to about 20 millimeters. The shape of the addresses may also enhance visual observation of the spectral response. For example, the addresses may be in the form of stripes, bands, dots, or any other geometric shape. The addresses may also be spaced apart a certain distance to provide a more visible spectral response. The spacing between two or more individual array addresses may, for example, range from about 0.01 to about 100 millimeters, in some embodiments from about 0.1 to about 50 millimeters, and in some embodiments, from about 1 to about 20 millimeters. The overall pattern of the array may take on virtually any desired appearance.

The array of indicators may be utilized in a variety of ways to provide information regarding an upper respiratory condition. In one embodiment, for example, a test sample may be obtained from the upper respiratory tract of a patient with any known sample collection device, such as with a swab, stick, syringe, etc. Once obtained, the sample may then be contacted with a test strip having an array of indicators. If desired, the sample collection device and test strip may be provided together in the form of a diagnostic test kit that may include other items, such as instructions, control strips, pretreatment solution, etc. For example, a pretreatment solution may be employed that contains a surfactant, such as described above, as a wetting agent for the sample.

Referring to Fig. 1A, for example, one embodiment of the present invention is shown in which an array 181 is formed on a substrate 180. The array 181 includes a broad spectrum indicator 183 (e.g., Reichardt's dye). When the indicator 183 undergoes a color change (Fig. 1B), the user is then alerted to the presence of bacteria in the sample. Likewise, when the broad spectrum indicator 183 remains substantially the same or undergo only a faint color change (Fig. 1C), the user is then alerted that the sample may contain other pathogens (e.g., viruses) or that the symptoms are due to other causes, such as allergies. If it is desired to further differentiate the type of bacteria present, the array 181 may also employ a first address 185 that undergoes a distinct color change in the presence of a specific type of bacteria. For example, the first address 185 may contain an indicator that undergoes a spectral response in the presence of gram-negative bacteria that is different than its spectral response in the presence of gram-positive bacteria. Other addresses may also be employed to help further identify the type of bacteria present. If it is desired to further differentiate viruses that may be present, the array 181 may also employ a second address 187 that undergoes a distinct color change in the presence of specific types of viruses. For instance, the second address 187 may contain an indicator that undergoes a spectral response in the presence of Rhinoviruses that is different than its response in the presence of Influenza or Human Parainfluenza viruses. It should be understood that separate addresses need not be employed in the present invention, and that when coupled with the information provided by the broad spectrum indicator, a single address may be sufficient. For example, the broad spectrum indicator may undergo a color change, thereby suggesting the presence of bacteria in the sample. With this information, the single address may then be observed to assess the presence of gram-negative or gram-positive bacteria.

Regardless, the spectral response of the indicator(s) may provide information about the presence of microorganisms to which it is exposed. If desired, the response of the indicator(s) (or array of indicators) may be compared to a control indicator (or array of indicators) formed in a manner that is the same or similar to the test indicator(s) with respect to microorganism responsiveness. The comparison may be made visually or with the aid of an instrument. Multiple control indicators may likewise be employed that correspond to different types of microorganisms at a certain concentration. Upon comparison, the microorganism may be identified by selecting the control indicator having a spectral response that is the same or substantially similar to the response of the test indicator, and then correlating the selected control to a particular microorganism or class of microorganisms.

As a result of the present invention, it has been discovered that the presence of bacteria, viruses, or other microorganisms may be readily detected through the use of indicators that undergoes a detectable color change. The color change is rapid and may be detected within a relatively short period of time. For example, the change may occur in about 30 minutes or less, in some embodiments about 10 minutes or less, in some embodiments about 5 minutes or less, in some embodiments about 3 minutes or less, and in some embodiments, from about 10 seconds to about 2 minutes. In this manner, the indicator may provide a "real-time" indication of the presence or absence of microorganisms. Such a "real time" indication may alert a user or caregiver to seek treatment (e.g., antibiotic). On the other hand, the lack of a certain color change may provide the user or caregiver with an assurance that the sample is free of infection.

The present invention may be better understood with reference to the following examples.

### EXAMPLES

### Materials Employed

All reagents and solvents were obtained from Sigma-Aldrich Chemical Company, Inc. of St. Louis, Missouri unless otherwise noted and were used without further purification. The microorganisms used in the study were:
1. Gram negative (viable)
   - *Escherichia coli* (ATCC #8739) *(E. coli)*
   - *Psuedomonas aeruginosa* (ATCC #9027) *(P. aeruginosa)*
   - *Salmonella choleraesuis* (Gibraltar Laboratories) *(S. choleraesuis)*
   - *Haemophilus influenzae* (ATCC # 49247 ) *(H. influenzae)*
   - *Moraxella lacunata* (ATCC # 17972 ) *(M. lacunata)*
2. Gram positive (viable)
   - *Staphylococcus aureus* (ATCC #6538) *(S. aureus)*
   - *Bacillus anthracis* (Gibraltar Laboratories) *(A. bacillus)*
   - *Streptococcus pyogenes* (ATCC # 10782) *(S. pyogenes)*
   - *Streptococcus pneumoniae* (ATCC # 10015) *(S. pneumoniae)*
3. Yeast (viable)
   - *Candida albicans* (ATCC #10231) *(C. albicans)*
4. Mold (viable)
   - *Aureobasidium pullulans* (ATCC # 16622) *(A. pullulans)*
   - *Penicillium janthinellum* (ATCC # 10069) *(P. janthinellum)*
5. Viruses (viable) (Gibraltar Laboratories)
   - *Herpes Simplex Virus 1 (HSV-1)* (ATCC # VR-260)
   - *Herpes Simplex Virus 2 (HSV-2)* (ATCC # VR-734)
   - *Adenovirus Type 2 (Adeno* 2) (ATCC # VR-846)
   - *Adenovirus Type 5 (Adeno* 5) (ATCC # VR-5)
   - *Coronavirus* (ATCC # VR-740)
   - *Rhinovirus Type* 42 (Received 5/13/82 from Hoffman La Roche)
   - *Influenza A (H2N2)* (ATCC # VR-1 00)
   - *Influenza A* (ATCC # VR-544)
   - *Parainfluenza 1 (Sendai)* (ATCC # VR-105)
   - *Influenza Avian* (ATCC # VR-797)

All Influenza strains were grown in chick embryos, and the rest of the viruses, with the exception of Coronavirus, utilized VERO-Kidney cells from the African Green Monkey as a host. Coronavirus was grown in WI-38 human diploid cells derived from female lung tissue. Dulbecco's Modified Eagle's Medium (DMEM) with 5% Fetal Bovine Serum (FBS) was used as the culture and dilution medium for all non-chick embryo viruses. Chorioallantoic fluid (CAF) was used for viruses grown up in the chick embryo system.

The indicators used in the study are listed with their molecular structure in Table 1:

**Table 1: Exemplary Indicators and Their Corresponding Structure**

| Indicator | Structure |
|---|---|
| 4-[(1-Methyl-4(1*H*)-pyridinylidene)ethylidene]-2,5-cyclohexadien-1-one hydrate | 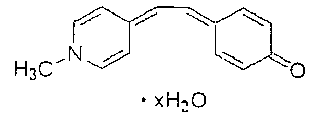 |
| 3-Ethyl-2-(2-hydroxy-1-propenyl)benzothiazolium chloride | 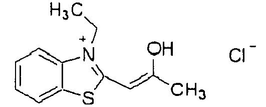 |
| 1-Docosyl-4-(4-hydroxystyryl)pyridinium bromide | 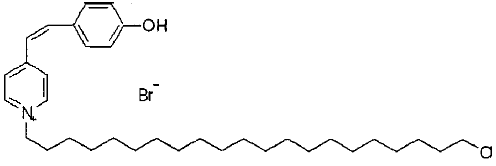 |
| *N,N*-Dimethylindoaniline | 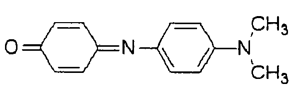 |
| Quinalizarin | 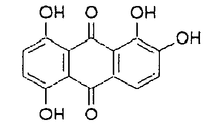 |
| Merocyanine 540 | 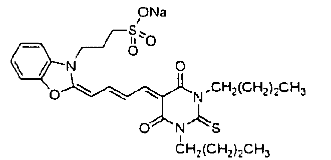 |
| Eriochrome Blue SE | 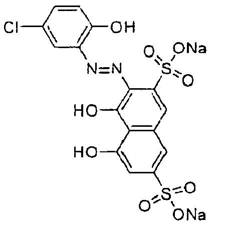 |
| Phenol Red | 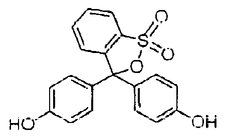 |
| Nile Blue A | 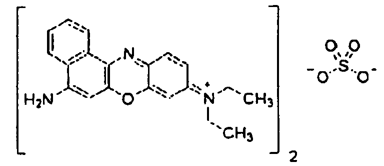 |
| 1-(4-Hydroxyphenyl)-2,4,6-triphenylpyridinium hydroxide inner salt hydrate | 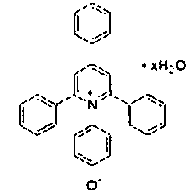 |
| Azomethine-H monosodium salt hydrate | 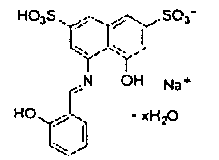 |
| Indigo carmine | 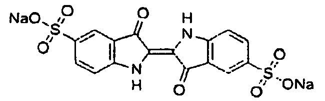 |
| Methylene Violet | 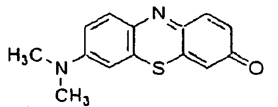 |
| Eriochrome Blue Black B | 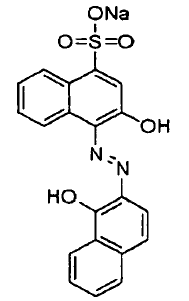 |
| Methylene Blue | 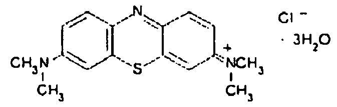 |
| Nile Red | 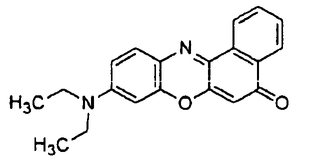 |
| Trypan Blue | 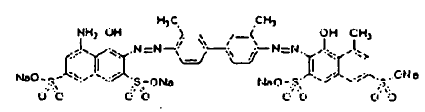 |
| Safranin O | 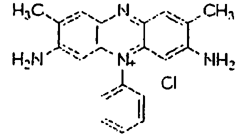 |
| Crystal Violet | 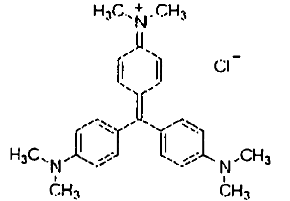 |
| Methyl Orange | 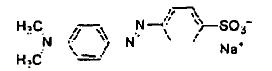 |
| Chrome Azurol S | 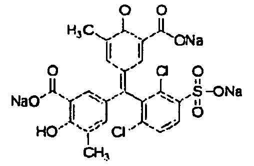 |
| Leucocrystal violet | 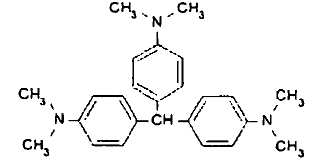 |
| Leucomalachite Green | 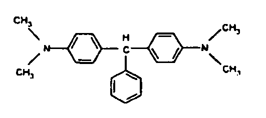 |
| Leucoxylene cyanole FF | 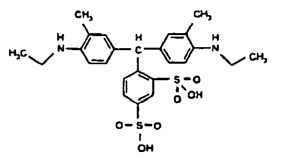 |
| 4,5-Dihydroxy-1,3-benzenedisulfonic acid disodium salt monohydrate | 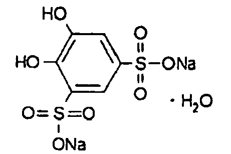 |
| 5-Cyano-2-[3-(5-cyano-1,3-diethyl-1,3-dihydro-2*H*-benzimidazol-2-ylidene)-1-propenyl]-1-ethyl-3-(4-sulfobutyl)-1*H*-benzimidazolium hydroxide inner salt | 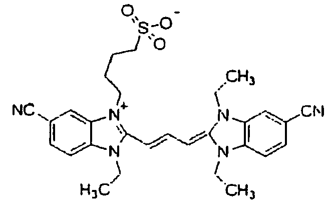 |
| Acid Green 25 | 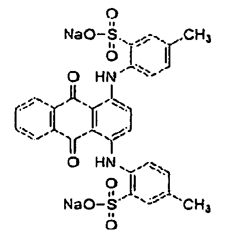 |
| Bathophenanthrolinedisulfonic acid disodium salt trihydrate | 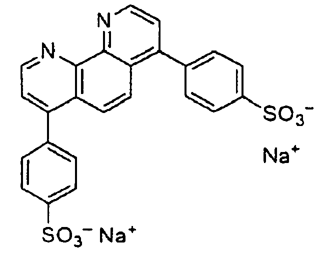 |
| Carminic Acid | 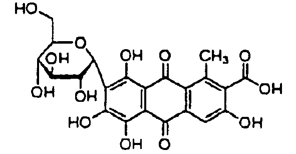 |
| Celestine Blue | 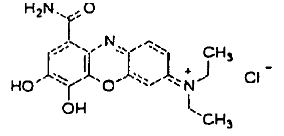 |
| Hematoxylin | 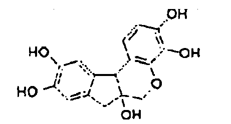 |
| Bromophenol Blue | 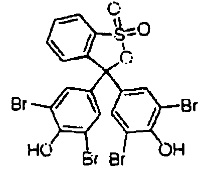 |
| Bromothymol blue | 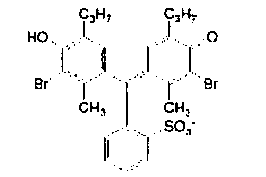 |
| Rose Bengal | 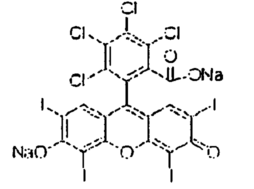 |
| Universal indicator 0-5 | Not available |
| Universal indicator 3-10 | Not available |
| Alizarin Complexone | 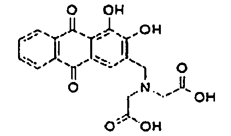 |
| Alizarin Red S | 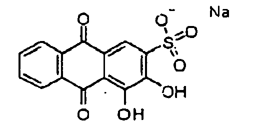 |
| Purpurin | 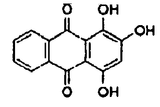 |
| Alizarin | 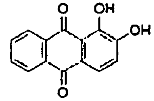 |
| Emodin | 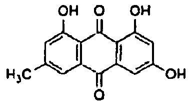 |
| Amino-4-hydroxyanthraquinone | 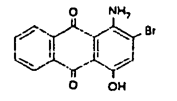 |
| Nuclear Fast Red | 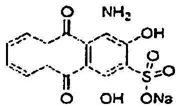 |
| Chlorophenol Red | 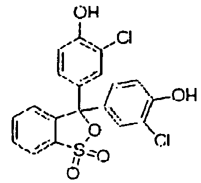 |
| Remazol Brilliant Blue R | 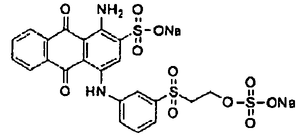 |
| Procion Blue HB | 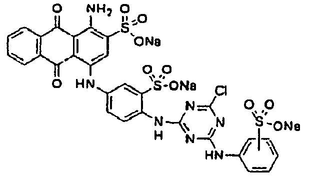 |
| Phenolphthalein | 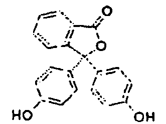 |
| Ninhydrin | 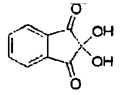 |
| Nitro blue tetrazolium | 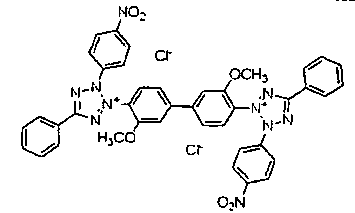 |
| Orcein | 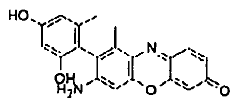 |
| Celestine blue | 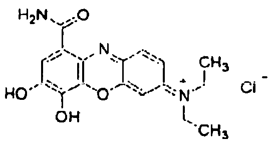 |
| Tetra Methyl-para-phenylene diamine (TMPD) | 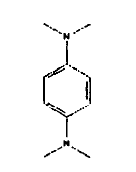 |
| 5,10,15,20-Tetrakis(pentafluorophenyl)porph yrin iron(III) chloride | 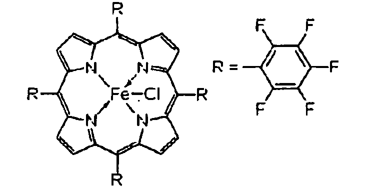 |

### EXAMPLE 1

Various indicators were tested for their ability to undergo a color change in the presence of *S. aureus*, *E. coli*, and *C. albicans* microorganisms. The indicators tested were Reichardt's dye, 1-Docosyl-4-(4-hydroxystyryl)pyridinium bromide, 3-Ethyl-2-(2-hydroxy-1-propenyl)-benzothiazolium chloride, 4-[(1-Methyl-4(1H)-pyridinylidene)ethylidene]-2,5-cyclohexadien-1-one hydrate, N,N-Dimethylindoaniline, Quinalizarin, Merocyanine 540, Eriochrome^{®} Blue SE (Plasmocorinth B), Phenol Red, Nile Blue A, 1-(4-Hydroxyphenyl)-2,4,6-triphenylpyridinium hydroxide inner salt hydrate, Azomethine-H monosodium salt hydrate, Indigo Camine, Methylene Violet, Eriochrome® Blue Black B, Biebrich scarlet-acid fuchsin solution, Methylene Blue, Nile Red, Trypan Blue, Safranin O, Crystal Violet, Methyl Orange, and Chrome Azurol S.

Unless otherwise specified, the indicators were dissolved in dimethylformamide (DMF). The indicator solutions were then pipetted onto 15-cm filter paper (available from VWR International - Catalog No. 28306-153) and allowed to dry. The filter paper was sectioned into quadrants to test four (4) samples - i.e., *S. aureus*, *E. coli*, *C. albicans*, and sterile water. 100 microliters of 10⁷ CFU/mL of *S. aureus* was pipetted onto the filter paper in one quadrant, 100 microliters of 10⁷ CFU/mL of *E. coli* was pipetted onto the filter paper in a second quadrant, 100 microliters of 10⁶ CFU/mL of *C. albicans* was pipetted onto the filter paper in a third quadrant, and sterile water was pipetted in the final quadrant. Color changes in the indicators were observed and recorded for each of the samples tested. The color was recorded immediately after the color change to inhibit the fading (or loss of intensity) of the colors as the samples dried. Table 2 presents the observations from the experiment.

**Table 2. Observations of Indicator Color Change (Group 1)**

| Indicator | Initial Color | Color Change w/ *S. aureus* | Color Change w/ *E. coli* | Color Change w/ *C. albicans* | Color Change w/ sterile water |
|---|---|---|---|---|---|
| Reichardt's dye | Blue | Colorless | Colorless | Colorless | No change |
| 1-Docosyl-4-(4-hydroxystyryl)pyridinium bromide | Yellow | Very faint orange | Faint orange | Faint orange | Very faint orange |
| 3-Ethyl-2-(2-hydroxy-1-propenyl)benzothiazolium chloride, | White / cream | No change | No change | No change | No change |
| 4-[(1-Methyl-4(1H)-pyridinylidene)ethylidene]-2,5-cyclohexadien-1-one hydrate | Bright yellow | No change | No change | No change | No change |
| N,N-Dimethylindoaniline | Grey | Faint pink | Very faint pink | Very faint pink | No change |
| Quinalizarin | Peach | Yellow | Faint purple | Purple | No change |
| Merocyanine 540 | Hot pink | Light purple | Yellowish pink | Deeper yellowish pink | Reddish pink |
| Eriochrome Blue SE (Plasmocorinth B) | Deep pink | Very faint purple | Purple | Deep purple | Lighter pink with dark pink border (dissolution) |
| Phenol Red | Yellow | Yellow with orange border | Orange | Deep red/orange | Green with orange border |
| Nile Blue A | Blue | Pink | Pink | Pink | No change |
| 1-(4-Hydroxyphenyl)-2,4,6-triphenylpyridinium hydroxide inner salt hydrate | Yellow | No change | No change | No change | No change |
| Azomethine-H monosodium salt hydrate | Yellow/ peach | Lighter with deeper border (dissolution) | Lighter with deeper border (dissolution) | Lighter with deeper border (dissolution) | Lighter with deeper border (dissolution) |
| indigo Carmine | Light blue | Deeper light blue | Deeper light blue | Deeper light blue | Light blue with deeper border (dissolution) |
| Methylene Violet | Deep blue/ violet | Deeper blue | Deeper blue | Deeper blue | No change |
| Eriochrome® Blue Black B | Dark muddy purple | Lighter muddy purple | Deep purple | Deep blue | Darker muddy purple |
| Biebrich scarlet-acid fuchsin solution | Bright red | Lighter with deeper border (dissolution) | Lighter with deeper border (dissolution) | Lighter with deeper border (dissolution) | Lighter with deeper border (dissolution) |
| Methylene Blue* | Bright blue | No change | No change | No change | No change |
| Nile Red | Bright purple | Light pink | Light pink | Light pink | Faint pink |
| Trypan Blue* | Deep blue | No change | No change | No change | Faintly lighter with deeper border (dissolution) |
| Safranin O | Bright salmon | Yellowish with salmon edge | Yellowish with salmon edge | Yellowish with salmon edge | Pinkish with salmon edge |
| Crystal Violet | Deep blue | No change | No change | No change | Faintly lighter with deeper border (dissolution) |
| Methyl Orange | Bright orange | Yellow | Yellow | Yellow | Lighter orange with dark orange border (dissolution) |
| Chrome Azurol S | Pink | Light orange with dark orange border | Light yellow with dark pink border | Brighter yellow with dark pink border | Light pink with dark pink border |

| | | | | | |
|---|---|---|---|---|---|
| * Dissolved in water | | | | | |

With the exception of Methyl Orange, Nile Red, and Merocyanine 540, the observed color change was almost immediate (1 to 2 minutes).

### EXAMPLE 2

Various indicators were tested for their ability to undergo a color change in the presence of *S. aureus*, *E. coli*, and *C. albicans* microorganisms. The indicators tested were Leucocrystal Violet, Leucomalachite Green, Leuco xylene cyanole FF, 4,5-Dihydroxy-1,3-benzenedisulfonic acid disodium salt monohydrate, 5-Cyano-2-[3-(5-cyano-1,3-diethyl-1,3-dihydro-2*H*-benzimidazol-2-ylidene)-1-propenyl]-1-ethyl-3-(4-sulfobutyl)-1*H*-benzimidazolium hydroxide inner salt, Acid Green 25, Bathophenanthrolinedisulfonic acid disodium salt trihydrate, Carminic Acid, Celestine Blue, Hematoxylin, Bromophenol Blue, Bromothymol Blue, Rose Bengal, Universal Indicator (0-5), and Universal Indicator (3-10). Unless otherwise specified, the indicators were dissolved in dimethylformamide (DMF). The VWR filter paper and indicators were prepared as described in Example 1. Table 3 presents the observations from the experiment.

**Table 3. Observations of Indicator Color Change (Group 2)**

| Indicator | Initial Color | Color Change w/ *S. aureus* | Color Change w/ *E. coli* | Color Change w/ *C. albicans* | Color Change w/ sterile water |
|---|---|---|---|---|---|
| Leucocrystal violet | White | Blue | Blue | Blue | No change |
| Leucomalachite Green | White | Green | Green | Green | No change |
| Leuco xylene cyanole FF | White | No change | No change | No change | No change |
| 4,5-Dihydroxy-1,3-benzenedisulfonic acid disodium salt monohydrate* | White | No change | No change | No change | No change |
| 5-Cyano-2-[3-(5-cyano-1,3-diethyl-1,3-dihydro-2*H* benzimidazol-2-ylidene)-1-propenyl]-1-ethyl-3-(4-sulfobutyl)-1*H*-benzimidazolium hydroxide inner salt | Bright reddish pink | Dark pink | Dark purplish pink | Dark greenish pink | Lighter pink with dark pink border (dissolution) |
| Acid Green 25 | Green | Lighter green with darker green border (dissolution) | Lighter green with darker green border (dissolution) | lighter green with darker green border (dissolution) | Lighter green with darker green border (dissolution) |
| Bathophenanthrolinedisulfonic acid disodium salt trihydrate** | White | No change | No change | No change | No change |
| Carminic Acid* | Reddish peach | Pale purple | Purple | Dark purple | Lighter peach with darker peach border (dissolution) |
| Celestine Blue | Dark lavender | Blue | Blue | Blue | Blue |
| Hematoxylin | Pale yellow | No change | Light purple | Darker purple | Pale yellow with darker yellow border (dissolution) |
| Bromophenol Blue | Bright Yellow | Dark blue | Dark blue | Dark blue | Lighter yellow with orangeish border (dissolution) |
| Bromothymol Blue | Yellow | Lighter yellow with darker yellow border | Light green | Darker green | Very light Yellow/whitish with darker yellow border |
| Rose Bengal | Hot pink | Darker pink | Purplish pink | Reddish pink | White with dark pink border (dissolution) |
| Universal Indicator (0-5) | Yellowish green | Yellowish blue | Yellowish blue | Yellowish blue | Lighter green with dark green border (dissolution) |
| Universal Indicator (3-10) | Peach | Pinkish peach | Orange-ish yellow | Yellow | Dark peach |

| | | | | | |
|---|---|---|---|---|---|
| * Dissolved in water ** Dissolved in DMF and water | | | | | |

With the exception of Leucocrystal Violet, Leucomalachite Green, and Leuco xylene cyanole FF, the observed color change was almost immediate (1 to 2 minutes).

### EXAMPLE 3

Various indicators were tested for their ability to undergo a color change in the presence of *S. aureus*, *E. coli*, and *C. albicans* microorganisms. The indicators tested were Alizarin Complexone, Alizarin Red S, Purpurin, Alizarin, Emodin, Amino-4-hydroxyanthraquinone, Nuclear Fast Red, Chlorophenol Red, Remazol Brilliant Blue R, Procion Blue HB, Phenolphthalein, tetraphenylporphine, tetra-o-sulphonic acid, and Ninhydrin. Unless otherwise specified, the indicators were dissolved in dimethylformamide (DMF). The VWR filter paper and indicators were prepared as described in Example 1. Table 4 presents the observations from the experiment.

**Table 4. Observations of Indicator Color Change (Group 3)**

| Indicator | Initial Color | Color Change w/ *S. aureus* | Color Change w/ *E. coli* | Color Change w/ *C. albicans* | Color Change w/ sterile water |
|---|---|---|---|---|---|
| Alizarin complexone | Yellow | Brown | Reddish purple | Purple | No change |
| Alizarin Red S | Yellow | Orangeish brown | Pinkish purple | Purple | Lighter yellow with darker yellow border (dissolution) |
| Purpurin | Peachish orange | Darker peachish orange | Reddish pink | Deeper reddish pink | Yellowish peach |
| Alizarin | Butter yellow | No change | Light brown | Purplish brown | Greenish butter yellow |
| Emodin | Yellow | No change | Faint Greenish orange | Deeper greenish orange | Greenish yellow |
| Amino-4-hydroxyanthraquinone | Pink | Lighter pink | Slightly lighter pink | Faintly lighter pink | Darker pink |
| Nuclear Fast Red | Reddish pink | Deeper reddish pink | Yellowish pink | Yellowish pink | Dark pink |
| Chlorophenol Red | Orange-ish yellow | Brown | Deep reddish purple | Deeper reddish purple | lighter orangish yellow with darker border (dissolution) |
| Remazol Brilliant Blue R | Bright blue | Lighter blue with dark blue border (dissolution) | Lighter blue with dark blue border (dissolution) | Lighter blue with dark blue border (dissolution) | Lighter blue with dark blue border (dissolution) |
| Procion Blue HB | Teal green | No change | No change | Faintly darker teal | Lighter teal with darker border (dissolution) |
| Phenolphthalein | White | No change | No change | No change | No change |
| Tetraphenylporphine, tetra-o-sulphonic acid | Black | Grey with darker borders (dissolution) | Grey with darker borders (dissolution) | Grey with darker borders (dissolution) | Grey with darkers borders (dissolution) |
| Ninhydrin | White | Deep purple | Deep purple | Slightly lighter deep | No change |

The observed color change was almost immediate (1 to 2 minutes).

### EXAMPLE 4

The ability to rapidly detect various gram-positive and gram-negative microorganisms utilizing the indicators of Examples 1-3 was demonstrated. Additional indicators were also tested, including Plasmocorinth B, Nitro Blue, Alizarin Complexone, Orcein, Tetra Methyl-para-phenylene diamine (TMPD), Nile Red, Eriochrome Blue Black B, Phenol Red, Alizarin Red S, Carminic Acid, Fe(III)C₃, Celestine Blue, Kovac's Reagent, Chrome Azurol S, Universal Indicator 3-10, Methyl Orange, Merocyanine 540, and Iron III Chloride Porphyrin. The gram-positive microorganisms tested were *S. aureus*, *L. acidophilus*, *S. epidermidis*, *B. subtilis*, and *E. faecalis*. The gram-negative microorganisms tested were *E. coli*, *P. aeruginosa*, *K. pneumoniae*, and *P. mirabilis*.

The indicator samples were prepared in a manner similar to Example 1. Unless otherwise specified, the indicators were dissolved in dimethylformamide (DMF). Each of the indicator solutions were pipetted onto two separate pieces of VVVR filter paper and allowed to dry. One filter paper sample with the dried indicator was sectioned into five approximately equal sections to test the five gram-positive microorganisms. The other filter paper sample was sectioned into quadrants to test the four gram negative microorganisms. 100 microliters of 10⁷ CFU/mL of each microorganism sample was pipetted into their respective section of the sample of filter paper. Table 5 presents the observations from the gram positive microorganisms and Table 6 presents the observations from the gram negative microorganisms.

**Table 5. Color Change Observations for Gram Positive Microorganisms**

| Indicator | Initial Color | Color Change w/ *B. subtilis* | Color Change w/*S. aureus* | Color Change w/ *S*. *epidermidis* | Color Change w/ *E. faecalis* | Color Change w/ *L*. *acidophilus* |
|---|---|---|---|---|---|---|
| Plasmocorinth B | Deep pink | Purplish pink | Very faint purplish pink | Deeper pink | Reddish pink | Deeper reddish pink |
| Nitro Blue Tetrazolium | Yellowish white | No change | No change | No change | No change | No change |
| Alizarin Complexone | Yellow | Brownish red | Lighter brownish red | Lighter brownish red | Lighter red | Brownish yellow |
| Orcein | Muddy purple | Light purple | Lighter muddy Purple | Darker muddy purple | Darker muddy purple | Darker muddy purple |
| Tetra Methyl-para-phenylene diamine (TMPD)* | Bright lavender | Colorless | Colorless | Not tested | Not tested | Colorless |
| Nile Red | Bright purple | Light pink | Light pink | Light pink | Light pink | Light pink |
| Eriochrome Blue Black B | Dark Muddy purple | Bluish purple | Lighter muddy purple | Darker muddy purple | Darker muddy purple | Darker muddy purple Purple |
| Phenol Red | Yellow | Orange with yellowish center | Yellow with orange border | Yellow with orange border | Yellow with orange border | Greenish yellow with orange border |
| Alizarin Red S | Yellow | Brownish pink | Light brown | Light brown | Light brown | Light Greenish brown |
| Carminic Acid* | Reddish peach | Pale purple | Paler purple | Paler purple | purplish peach | Yellowish peach |
| Fe(III)C₃ | White | No change | No change | Not tested | Not tested | No change |
| Celestine Blue | Dark lavender | Blue | Blue | Blue | Blue | Blue |
| Kovac's Reagent | Pale yellow | White with greenish center and yellow border | White with greenish center and yellow border | White with greenish center and yellow border | White with greenish center and yellow border | White with greenish center and brown border |
| Chrome Azurol S | Pink | Pale yellow with reddish border | Light orange with dark orange border | Light yellowish orange with dark orange border | Light orange with dark orange border | Light red with dark red border |
| Universal Indicator 3-10 | Peach | Lighter peach with yellow center | Lighter peach Lighter peach center | Lighter peach with yellow center | Lighter peach | Red |
| Methyl Orange | Bright orange | Yellow | Yellow | Yellow | Yellow | Yellow |
| Merocyanine 540 | Hot pink | Light purple | Light purple | Light purple | Light purple | Light purple |
| Iron III Chloride Porphyrin* | Light mustard yellow | Darker mustard yellow | Darker mustard yellow | Darker mustard yellow | mustard yellow | mustard yellow |

| | | | | | | |
|---|---|---|---|---|---|---|
| *Dissolved in water | | | | | | |

**Table 6. Color Change Observations for Gram Negative Microorganisms**

| Indicator | Initial Color | Color Change w/ *E. coli* | Color Change w/*P. aeruginosa* | Color Change w/ *K*. *pneumoniae* | Color Change w/ *P. mirabilis* |
|---|---|---|---|---|---|
| Plasmocorinth B | Deep pink | Light purple | Deep blue | pink reddish | Deep reddish pink |
| Nitro blue tetrazolium | Yellowish white | No change | No change | No change | No change |
| Alizarin Complexone | Yellow | Purple | Deeper purple | Brownish purple | Purple |
| Orcein | Muddy purple | Light purple | Dark purple | Brownish purple | Darker brownish purple |
| Tetra Methyl-para-phenylene diamine (TMPD)* | Bright lavender | Colorless | Dark purple | Colorless | Colorless |
| Nile Red | Bright purple | Light pink | Light pink | Light pink | Light pink |
| Eriochrome Blue Black B | Dark Muddy purple | Bluish purple | Dark blue | Darker purple | Darker purple |
| Phenol Red | Yellow | Orange | Dark red/orange | orange border orange border | Orange |
| Alizarin Red S | Yellow | Brownish purple | Deep reddish purple | Light brownish purple | Deep reddish purple |
| Carminic Acid* | Reddish peach | Blueish purple | Dark purple | Paler Bluish purple | Purple |
| Fe(III)C₃ | White | No change | No change | Not tested | No change |
| Celestine Blue | Dark | Blue | Blue | Blue | Blue |
| | | | | | |

| Indicator | Initial Color | Color Change w/ *E. coli* | Color Change w/ *P*. *aeruginosa* | Color Change w/ *K. pneumoniae* | Color Change w/ *P. mirabilis* |
|---|---|---|---|---|---|
| Kovac's Reagent | Pale yellow | White with greenish center and yellow border | White with greenish center and yellow and yellow border | White with greenish center and yellow border | White with greenish center and yellow border |
| Chrome Azurol S | Pink | Greenish yellow with border | Bright yellow with dark pink border | Greenish yellow with dark pink border | Greenish yellow with dark pink border |
| Universal Indicator 3-10 | Peach | Lighter Peach with yellow center | Light green Light green | Darker peach with yellow center | Lighter peach with yellow center |
| Methyl Orange | Bright Orange | Yellow | Yellow | Yellow | Orange/ yellow |
| Merocyanine 540 | Hot pink | Yellowish pink | Yellowish pink | Yellowish pink | Yellowish pink |
| Iron III Chloride Porphyrin* | Mustard yellow | Darker mustard yellow | Darker mustard yellow | Darker mustard yellow | Darker mustard yellow |

| | | | | | |
|---|---|---|---|---|---|
| *Dissolved in water | | | | | |

With the exception of Methyl Orange, Nile Red, Tetra Methyl-para-phenylene diamine (TMPD), and Merocyanine 540, the observed color change was also most immediate (1 to 2 minutes).

### EXAMPLE 5

The ability to rapidly detect upper respiratory bacterial pathogens utilizing a group of indicators was demonstrated. The indicators tested were Alizarin Red S, Universal Indicator 3-10, Nile Red, Plasmocorinth B, Iron III Porphyrin, Eriochrome Blue Black B, Chrome Azurol S, Orcein, Alizarin Complexone, Phenol Red, Carminic Acid, Methyl Orange, and TMPD. The upper respiratory infection pathogens tested were *H. influenzae, M. lacunata, S. pyogenes, S. pneumoniae, A. pullulans,* and *P. janthinellum.* The indicator samples were prepared in a manner similar to Example 1. Unless otherwise specified, the indicators were dissolved in dimethylformamide (DMF). Color changes in the indicators were observed and recorded for each of the samples tested. Table 7 presents the observations from the upper respiratory infection pathogens.

**Table 7. Color Change for Upper Respiratory Infection Pathogens**

| Indicator | Initial Color | Color Change w/ H. *influenzae* | Color Change w/ *M. lacunata* | Color Change w/ S. *pyogenes* | Color Change w/ *S*. *pneumomae* | Color Change w/ *A. pullulans* | Color change w/ *P*. *janthinellum* |
|---|---|---|---|---|---|---|---|
| Alizarin Red S | Dark mustard yellow | Red | Brownish red | Light brown | Light brown | Bright brownish yellow | Bright brownish yellow |
| Universal Indicator 3-10 | Dark peach | Greenish yellow | Greenish yellow | Brownish yellow | Brownish yellow | Darker peach | Darker peach |
| Nile Red | Bright purple | Pink | Pink | Pink | Pink | Dark pink | Dark pink |
| Plasmocorinth B | Bright pink | Bluish purple Bluish purple | Darker bluish purple | Dark pink Dark pink | Dark pink Dark pink | Lighter bright pink | Lighter bright pink |
| Iron III Porphyrin* | Mustard yellow | Darker mustard yellow | Darker mustard yellow | Darker mustard yellow | Darker mustard yellow | Darker mustard yellow | Darker mustard yellow |
| Eriochrome Blue Black B | Grape | Dark blue | Dark blue | Dark grapish pink | Dark grapish pink | Dark grape | Dark grape |
| Chrome Azurol S | Light orange | Light green with dark red border | Light green with dark red border | Brownish red with dark red border | Brownish red with dark red border | Light pink with dark red border | Light pink with dark red border |
| Orcein | Muddy purple | Bight purple | Bright purple | Bluish muddy purple | Darker muddy purple | Lighter muddy purple | Lighter muddy purple |
| Alizarin Complexone | Yellow | Reddish purple | Purple | Brown | Brown | Yellow | Yellow |
| Phenol Red | Orangish yellow | Orangish red | Bright red | Greenish yellow | Greenish yellow | Bright yellow | Bright yellow |
| Carminic Acid* | Bright peach | Purple | Dark purple | Brownish / Purplish | Brownish purplish peach | Brighter peach | Brighter peach |
| Methyl Orange | Dark orange | Yellow | Yellow | Yellow | Yellow | Brownish yellow | Brownish yellow |
| TMPD* | Yellowish | White | Purple | Not tested | Pink | Not tested | Not tested |

| | | | | | | | |
|---|---|---|---|---|---|---|---|
| *Dissolved in water | | | | | | | |

With the exception of Methyl Orange, Nile Red, and tetramethyl-para-phenylene diamine (TMPD), the observed color change was almost immediate (1 to 2 minutes).

### EXAMPLE 6

Filter paper (available from VWR International) was treated with solutions of Chrome Azurol, Alizarin Complexone, Plasmocorinth B, and Phenol Red (all dissolved in DMF). The samples were hung dry to evaporate the solvent. Solutions of *C. albicans, E. coli,* and *S. aureus* were diluted in ten-fold dilutions using Trypticase Soybean Broth TSB) media, and is some cases, sterile water. Concentrations ranged from 10⁸ CFU/mL (stock solution) down to 10¹ CFU/mL for both *E. coli* and *S. aureus,* and 10⁷ CFU/mL (stock solution) down to 10¹ CFU/mL for *C. albicans.* TSB and water were used as control solutions. 100 µL aliquots of each solution were applied to the samples. The color changes are summarized in Tables 8-12.

**Table 8: Response to Dilutions of C. albicans in TSB media**

| Dye | Initial Color | 10⁶ CFU/ml | 10⁵ CFU/ml | 10⁴ CFU/ml | 10³ CFU/ml | 10² CFU/ml | 10¹ CFU/ml | TSB Control |
|---|---|---|---|---|---|---|---|---|
| Phenol Red | Bright yellow | orange | Slightly darker orange | Slightly darker orange | Slightly darker orange | Slightly darker orange | Slightly darker orange | Dark orange |
| Plasmocorinth B | Bright pink | Purplish blue | Slightly darker Purplish blue | Slightly darker Purplish blue | Slightly darker Purplish blue | Slightly darker Purplish blue | Slightly darker Purplish blue | Dark purplish blue |
| Alizarin Complexone | Bright yellow | Brownish purple | Slightly darker Brownish purple | Slightly darker Brownish purple | Slightly darker Brownish purple | Slightly darker Brownish purple | Slightly darker Brownish purple | Dark Brownish purple |
| Chrome Azurol | rose | Greenish yellow | Slightly darker Greenish yellow | Slightly darker Greenish yellow | Slightly darker Greenish yellow | Slightly darker Greenish yellow | Slightly darker Greenish yellow | Yellowish green |

**Table 9: Response to Dilutions of S. aureus in TSB media**

| Dye | Initial Color | 10⁸ CFU/ml (undiluted) | 10⁷ CFU/ml | 10⁵ CFU/ml | 10⁵ CFU/ml | 10⁴ CFU/ml | 10³ CFU/ml | 10² CFU/ml | TSB Control |
|---|---|---|---|---|---|---|---|---|---|
| Phenol Red | Bright yellow | Bright yellow | orange | Slightly darker orange | Slightly darker orange | Slightly darker orange | Slightly darker orange | Slightly darker orange | Dark orange |
| Plasmocorinth B | Bright pink | Bright purplish pink | Purplish blue | Slightly darker Purplish blue | Slightly darker Purplish blue | Slightly darker Purplish blue | Slightly darker Purplish blue | Slightly darker Purplish blue | Dark purplish blue |
| Alizarin Complexone | Bright yellow | Light brown | Brownish purple | Slightly darker Brownish purple | Slightly darker Brownish purple | Slightly darker Brownish purple | Slightly darker Brownish purple | Slightly darker Brownish purple | dark Brownish purple |
| Chrome Azurol | rose | Brownish yellow | Greenish yellow | Slightly darker Greenish yellow | Slightly darker Greenish yellow | Slightly darker Greenish yellow | Slightly darker Greenish yellow | Slightly darker Greenish yellow | Yellowish green |

**Table 10: Response to Dilutions of S. aureus in water**

| Dye | Initial Color | 10⁷ CFU/ml (in H₂O) | Water Control |
|---|---|---|---|
| Phenol Red | Bright yellow | N/A | Light yellow |
| Plasmocorinth B | Bright pink | Bright pink | Light pink |
| Alizarin Complexone | Bright yellow | Pale yellow | Pale yellow |
| Chrome Azurol | rose | Greenish red-pink | Light red-pink |

**Table 11: Response to Dilutions of E. coli in TSB media**

| Dye | Initial Color | 10⁸ CFU/ml (undiluted) | 10⁷ CFU/ml | 10⁶ CFU/ml | 10⁵ CFU/ml | 10⁴ CFU/ml | 10³ CFU/ml | 10² CFU/ml | TSB Control |
|---|---|---|---|---|---|---|---|---|---|
| Phenol Red | Bright yellow | Light orange | orange | Slightly darker orange | Slightly darker orange | Slightly darker orange | Slightly darker orange | Slightly darker orange | Dark orange |
| Plasmocorinth B | Bright pink | Pinkish purple | Purplish blue | Slightly darker Purplish blue | Slightly darker Purplish blue | Slightly darker Purplish blue | Slightly darker Purplish blue | Slightly darker Purplish blue | Dark purplish blue |
| Alizarin Complexone | Bright yellow | Purplish brown | Brownish purple | Slightly darker Brownish purple | Slightly darker Brownish purple | Slightly darker Brownish purple | Slightly darker Brownish purple | Slightly darker Brownish purple | dark Brownish purple |
| Chrome Azurol | rose | Light green | Greenish yellow | Slightly darker Greenish yellow | Slightly darker Greenish yellow | Slightly darker Greenish yellow | Slightly darker Greenish yellow | Slightly darker Greenish yellow | Yellowish green |

**Table 12: Response to Dilutions of E. coli in water**

| Dye | Initial Color | 10⁷ CFU/ml (in H₂O) | Water Control |
|---|---|---|---|
| Phenol Red | Bright yellow | Orangish yellow | Light yellow |
| Plasmocorinth B | Bright pink | Bright pink | Light pink |
| Alizarin Complexone | Bright yellow | Brownish yellow | Pale yellow |
| Chrome Azurol | rose | Dark green | Light red-pink |

Thus, a color change was observed for the microorganisms that was different than the media alone, although the difference was somewhat more subtle for the dilute solutions. Without intending to be limited in theory, it is believed that the more subtle difference for the dilute solutions was due in part to the lack of time given to the microorganisms to condition the media (the experiment was conducted shortly after dilution). In contrast, the stock solutions contained microorganisms that had been in the media for 24 hours.

### EXAMPLE 7

Select dyes were also tested with a group of viruses at Gibraltar Laboratories (Fairfield, NJ). The dyes employed are set forth below in Table 13.

**Table 13: Dyes for Virus Testing**

| **Code Number** | **Dye** |
|---|---|
| 1 | Chrome Azurol S |
| 2 | Erioglaucine |
| 3 | Hematoxylin |
| 4 | Alizarin Red S |
| 5 | Quinalizarin |
| 6 | TMPD |
| 7 | Bromophenol Blue |
| 8 | Plasmocorinth B |
| 9 | Chlorophenol Red |
| 10 | Eriochrome Blue Black B |
| 11 | Nile Blue A |
| 12 | Alizarin Complexone |
| 13 | Merocyanine 540 |
| 14 | Phenol Red |
| 15 | Bromothymol Blue |
| 16 | Alizarin |
| 17 | Fast Red AL Salt |
| 18 | Carminic Acid |
| 19 | Purpurin |
| 20 | Emodin |
| 21 | Neutral Red |
| 22 | 1,4-dihydroxyanthraquinone |
| 23 | Nuclear Fast Red |
| 24 | Universal Indicator Solution 3-10 |
| 25 | Kovac's Reagent |

The dyes were dissolved in DMF and coated onto twenty five filter paper samples. The samples were hung to dry and grouped into two sets and applied to two dye-treated filter paper samples for the study. For the first set, Herpes Simplex 1, Herpes Simplex 2, Adenovirus Type 2, Adenovirus Type 5, Coronavirus, and a DMEM control were tested on the first of each dye sample. For the second set, the remaining viruses, as well as a DMEM and CAF control were tested on the second dye sample. Testing was performed by taking a 50 µL aliquot of each virus or control and applying it to the sample. Photos were taken both immediately and after several minutes to document the color changes observed. The resulting color changes are summarized in Tables 14 and 15.

**Table 14: First Group of Viruses**

| Dyes Tested | Initial Color | Color Change w/ HSV-1 | Color Change w/ HSV-2 | Color Change w/ Adeno 2 | Color Change w/ Adeno 5 | Color Change w/ Coronavirus | Color change w/ DMEM |
|---|---|---|---|---|---|---|---|
| Chrome Azurol S | Yellow | Light greenish-purple | Light greenish-purple | Darker greenish-purple | Darker greenish-purple | Dark green | Dark green |
| Erioglaucine | Light turquoise | Lighter turquoise (dissolution) | Lighter turquoise (dissolution) | Lighter turquoise (dissolution) | Lighter turquoise (dissolution) | Lighter turquoise (dissolution) | Lighter turquoise (dissolution) |
| Hematoxylin | Pale yellow/ Peach | Brighter yellow (purplish) | Brighter yellow (purplish) | Brighter yellow (purplish) | Brighter yellow (purplish) | purple | Darker purple |
| Alizarin Red S | Yellow | Light red | Light red | Slightly darker light red | Light red | Purplish red | Darker purplish red |
| Quinalizarin | Peach | Yellowish peach | Yellowish peach | Darker yellowish peach | Yellowish peach | purple | Dark purple |
| TMPD | Light purple | colorless | colorless | colorless | colorless | colorless | colorless |
| Bromophenol Blue | Bright yellow | Dark purple | Dark purple | Dark purple | Dark purple | Darker purple | Darker purple |
| Plasmocorinth B | Bright pink | Lighter pink | Lighter pink | Lighter pink | Lighter pink | purplish | Darker purple |
| Chlorophenol Red | Yellow | lavender | lavender | Darker lavender | Light lavender | Dark pink purple | Darker pink purple |
| Eriochrome Blue Black B | Purplish-pink | Light purple | Light purple | Slightly darker purple | Slightly darker purplish pink | Dark bluish purple | Darker bluish purple |
| Nile Blue A (coating was non-uniform) | Dusty blue | purple | Slightly darker purple | purple | Slightly darker purple | Deeper purple | Deeper purple |
| Alizarin Complexone | Light yellow | Light lavender | lavender | lavender | lavender | purple | Purple |
| Merocyanine 540 | Hot pink | Pale pink | Pale pink | Pale pink | red | red | Darker red |
| Phenol Red | Orange | Bright yellow | Bright yellow | Bright yellow | Light neon green | Yellow with dark pink ring in center | Yellow with dark pink circle in center |
| Bromothymol Blue | Yellow | Pale yellow | Pale yellow | Pale yellow | Pale greenish yellow | green | Darker green |
| Alizarin | Pale yellow (almost beige) | lavender | lavender | Pale lavender | Muddy lavener | purple | Darker purple |
| Fast Red AL Salt | Pale yellow (almost beige) | No visible change | No visible change | No visible change | Wet spot (no visible color change) | Very pale lavender | Slightly darker pale lavender |
| Carminic Acid | Bright Orange-peach | Lighter orange peach | Lighter orange peach | Lighter orange peach | Darker orange peach | Light purple | Light purple |
| Purpurin | Dark beige | Pale lavender | Pale lavender | Slightly darker pale lavender | Pale lavender | Light purple | Light purple |
| Emodin | Yellow | Pale yellow | Pale yellow | Pale yellow | Pale yellow | Pale pinkish red | Slightly darker pinkish red |
| Neutral Red | Dusty pink | Faint yellow | Faint yellow | Brighter yellow | Yellowish pink | Greenish yellow | Greenish yellow |
| 1,4-dihydroxy-anthraquinone | Brownish yellow | Pale brownish yellow | Pale brownish yellow | Pale brownish yellow | Pale brownish yellow | Pale brownish yellow | Pale brownish yellow |
| Nuclear Fast Red | Pink | Lighter pink | Lighter pink | Darker pink | Darker pink | Darker pink | Darker pink |
| Universal Indicator Solution 3-10 | Bright peach | yellow | yellow | yellow | yellow | Bright greenish yellow | Bright greenish yellow |
| Kovac's Reagent | Pale green | White with bluish center | White with bluish center | White with bluish center | White with bluish center | White with bluish center | White with bluish center |

**Table 15: Second Group of Viruses**

| Dyes Tested | Initial Color | Color Change w/ Rhinovirus | Color Change w/ Influenza (Japan) | Color Change w/ Influenza (Hong Kong) | Color Change w/ Parainfluenza | Color Change w/ Influenza Avian | Color change w/ DMEM | Color change w/ CAF |
|---|---|---|---|---|---|---|---|---|
| Chrome Azurol S | Yellow | Greenish-purple | Greenish-purple | Greenish-purple | Greenish-purple | Darker Greenish-purple | Dark greenish-purple | Dark greenish-purple |
| Erioglaucine | Light turquoise | Lighter turquoise (dissolution) | Lighter turquoise (dissolution) | Lighter turquoise (dissolution) | Lighter turquoise (dissolution) | Lighter turquoise (dissolution) | Lighter turquoise (dissolution) | |
| Hematoxylin | Pale yellow/ Peach | Light purple/ lavender | Darker purple | Brownish purple | Darker purple | Brownish purple | Darker purple | Darker purple |
| Alizarin Red S | Yellow | Light reddish purple | Darker reddish purple | Darker reddish purple | Deep reddish purple | Darker reddish purple | Deep reddish purple | Deep reddish purple |
| Quinalizarin | Peach | Light purple | Dark purple | Brownish purple | Dark purple | Darker purple | Dark purple | Dark purple |
| TMPD | Light purple | colorless | colorless | colorless | colorless | colorless | colorless | colorless |
| Bromophenol Blue | Bright yellow | Deep purple | Deep purple | Deep purple | Deep purple | Deep purple | Deep purple | Deep purple |
| Plasmocorinth B | Bright pink | Light purple | purplish | Purplish (slightly darker than Japan strain) | purplish | Darker purplish (similar to Hong Kong) | purplish | Darker purple |
| Chlorophenol Red | Yellow | magenta | magenta | magenta | magenta | magenta | Slightly darker magenta | Slightly darker magenta |
| Eriochrome Blue Black B | Purplish-pink | Bluish purple | Dark bluish purple | Darker bluish purple | Darker bluish purple | Darker bluish purple | Deep bluish purple | Deep bluish purple |
| Nile Blue A (coating is very uneven) | Dusty blue | Bluish purple | purple | purple | purple | Slightly lighter purple | purple | Darker purple |
| Alizarin Complexone | Light yellow | Light purple | purple | purple | Slightly darker purple | Dark purple | Dark purple | Dark purple |
| Merocyanine 540 | Hot pink | red | Deeper red | Deeper red | Deeper red | Deeper red | Deeper red | Deep red |
| Phenol Red | Orange | Yellow with dark pink disc | Yellow with thinner dark pink disc | Yellow with very thin dark pink disc | Yellow with dark pink middle | Yellow with dark pink disk | Yellow with almost solid dark pink center | Yellow with dark pink disk (faint) |
| Bromothymol Blue | Yellow | Light green | Darker green | Darker green | Dark green | Dark green | Dark green | Dark green |
| Alizarin | Pale yellow (almost beige) | Light purple | Light purple | Darker purple | Light purple | Darker purple | Darker purple | Darker purple |
| Fast Red AL Salt | Pale yellow (almost beige) | No visible color change | No visible color change | No visible color change | No visible color change | No visible color change | No visible color change | No visible color change |
| Carminic Acid Carminic Acid | Orange-peach | purple | Purplish orange | Deeper purplish orange | Darker purple | Purplish orange | Darker purple | Darker purple |
| Purpurin | Dark beige | Light purple | Darker purple | Darker purple | Darker purple | Darker purple | Darker purple | Darker purple |
| Emodin | Yellow | Pale pinkish red | Pinkish red | Brownish pink | Pinkish red | Brownish red | Pinkish red | Brownish red |
| Neutral Red | Dusty pink | Mustard yellow | Darker mustard yellow | Greenish yellow | Darker mustard yellow | Greenish yellow | Greenish yellow | Greenish yellow |
| 1,4-dihydroxy-anthraquinone | Brownish yellow | Pale brownish yellow | Pale brownish yellow | Pale brownish yellow | Pale brownish yellow | Pale brownish yellow | Pale brownish yellow | Pale brownish yellow |
| Nuclear Fast Red | Pink | Lighter pink-red | Darker pink red | red | Darker pink red | red | red | Red |
| Universal Indicator Solution 3-10 | Bright peach | Bright yellow | Bright greenish yellow | yellow | Bright greenish yellow | Bright greenish yellow | Bright greenish yellow | Bright greenish yellow (more green) |
| Kovac's Reagent | Pale green | White with bluish center | White with bluish center | White with bluish center | White with bluish center | White with bluish center | White with bluish center | White with bluish center |

Digital photos were also analyzed using NIH Image (ImageJ) for intensity analysis. A list of the most interesting dyes and the relative intensities with each type of virus are given in Tables 16 and 17.

**Table 16: Mean Intensity Values for Select Dyes with Viruses**

| | **HSV-1** | **HSV-2** | **Adeno 2** | **Adeno 5** | **Coronavirus** | **DMEM** | **Rhinovirus** |
|---|---|---|---|---|---|---|---|
| **Chrome Azurol** S **(161.0)** | 143.4 | 145.4 | 128.6 | 134.5 | 119.4 | 116.9 | 140.8 |
| **Alizarin Red S (162.7)** | 135.9 | 139.4 | 123.1 | 131.0 | 100.6 | 99.3 | 130.1 |
| **Quinalizarin (155.5)** | 145.4 | 143.0 | 136.3 | 132.9 | 106.7 | 104.9 | 138.7 |
| **Plasmacorinth B (161.5)** | 141.9 | 139.4 | 138.5 | 136.7 | 119.0 | 106.3 | 156.2 |
| **Hematoxylin (176.7)** | 156.1 | 153.4 | 144.3 | 148.9 | 135.1 | 127.5 | 162.3 |
| **Eriochrome Blue Black B (152.6)** | 133.6 | 126.5 | 120.1 | 116.7 | 95.6 | 79.2 | 127.5 |
| **Alizarin Complexone (157.6)** | 120.9 | 126.8 | 124.9 | 122.7 | 102.0 | 94.5 | 137.7 |
| **Merocyanine 540 (149.0)** | 151.4 | 146.7 | 146.1 | 123.2 | 104.3 | 103.2 | 116.3 |
| **Bromothymol Blue (135.4)** | 127.5 | 136.7 | 120.7 | 111.9 | 74.2 | 72.6 | 109.9 |
| **Alizarin (160.0)** | 146.8 | 154.5 | 149.5 | 134.6 | 119.0 | 116.7 | 130.5 |
| **Purpurin (150.0)** | 141.0 | 128.9 | 131.3 | 124.1 | 110.3 | 103.9 | 145.1 |
| **Emodin (145.0)** | 133.5 | 126.1 | 127.5 | 120.8 | 108.9 | 106.2 | 133.1 |
| **Neutral Red (133.2)** | 118.6 | 119.1 | 105.2 | 108.0 | 96.0 | 94.6 | 127.7 |
| **Nuclear Fast Red (143.0)** | 132.9 | 138.2 | 115.0 | 125.7 | 104.7 | 109.8 | 120.6 |

**Table 17: Mean Intensity Values for Select Dyes with Viruses**

| | **Influenza (Japan)** | **Influenza (Hong Kong)** | **Influenza (Avian)** | **Parainfluenza** | **DMEM** | **CAF** |
|---|---|---|---|---|---|---|
| **Chrome Azurol S (161.0)** | 142.3 | 131.7 | 140.8 | 124.0 | 122.1 | 111.5 |
| **Alizarin Red** S **(162.7)** | 119.5 | 118.1 | 112.0 | 111.0 | 103.2 | 100.9 |
| **Quinalizarin (155.5)** | 125.2 | 116.8 | 113.5 | 111.6 | 104.3 | 99.0 |
| **Plasmocorinth B (161.5)** | 151.0 | 137.8 | 151.0 | 116.7 | 130.6 | 100.0 |
| **Hematoxylin (176.7)** | 154.6 | 153.3 | 151.4 | 146.8 | 144.3 | 137.0 |
| **Eriochrome Blue Black B (152.5)** | 110.8 | 104.9 | 81.2 | 92.5 | 74.5 | 54.2 |
| **Alizarin Complexone (157.6)** | 135.4 | 125.5 | 130.4 | 113.2 | 119.0 | 111.7 |
| **Merocyanine 540 (149.0)** | 120.2 | 105.9 | 114.6 | 101.4 | 104.8 | 88.3 |
| **Bromothymol Blue (135.4)** | 110.2 | 97.2 | 95.0 | 91.6 | 92.7 | 87.1 |
| **Alizarin (160.0)** | 130.7 | 117.7 | 130.9 | 122.6 | 121.8 | 111.1 |
| **Purpurin (150.0)** | 135.4 | 128.3 | 131.2 | 123.6 | 121.4 | 113.0 |
| **Emodin (145.0)** | 126.9 | 120.7 | 120.9 | 119.8 | 117.8 | 105.2 |
| **Neutral Red (133.2)** | 127.4 | 115.2 | 120.1 | 108.5 | 102.4 | 94.6 |
| **Nuclear Fast Red (143.0)** | 128.3 | 112.5 | 127.9 | 109.6 | 116.8 | 101.3 |

In general, color changes seemed to be split into two groups for each dye. The Herpes and Adeno viruses tended to produce a similar type of color change, while the Coronavirus, Rhinovirus, and Influenza viruses produced a similar type of color change as well. Interestingly, the Herpes and Adeno viruses are all comprised of double-stranded DNA, contain a lipid membrane, are all similar in size, and are icosohedral in shape. Coronavirus, Rhinovirus, and the various Influenza viruses all contain RNA, though the shape varies (helical for Influenza, icosohedral for Rhinovirus, and asymmetrical for Coronavirus). The pH values of these solutions also tended to group together as well. Though HSV-1, HSV-2, Adeno 2, Adeno 5, Coronavirus, and Rhinovirus were all cultured in DMEM (pH = 7.5), each organism-containing solution had a different pH, indicating that the organisms are secreting metabolites or other factors which influence affect their surroundings. A list of pH recordings is provided in Table 18 (taken using ColorpHast pH strips).

**Table 18: pH Values**

| **Solution** | **pH Value** |
|---|---|
| DMEM | 7.5 |
| HSV-1 | 6.5-7.0 |
| HSV-2 | 6.5-7.0 (closer to 6.5) |
| Adeno 2 | 6.5-7.0 |
| Adeno 5 | 5.5-6.0 |
| Coronavirus | 7.5-8.0 (closer to 8.0) |
| Rhinovirus | 7.5-8.0 (closer to 8.0) |
| CAF | 8.0 |
| Influenza A (Japan) | 7.5-8.0 (closer to 8.0) |
| Influenza A (Hong Kong) | 7.5-8.0 (closer to 8.0) |
| Parainfluenza | 7.5-8.0 (closer to 8.0) |
| Influenza Avian | 7.5-8.0 (closer to 8.0) |

Though pH appears to play a role in the color changes observed, it does not seem to be the only influencing factor. Many of the dyes tested are not known to be traditional pH indicators, but are affected by ions. Eriochrome Blue Black B, for instance, is a metal titration dye that is typically used at high pH values (around 10.0). The color changes from blue to red in the presence of the metal ions. For microbial detection, this dye is being used in its red state and a change to blue is observed in the presence of particular microbes. This effect is likely pH-dependent; however, there are distinctive differences between samples that have relatively similar pH values, such as Rhinovirus and Influenza viruses.

Intensity analysis using ImageJ confirmed that, in general, Rhinovirus tended to have color changes of lesser intensity than flu viruses. Based on the results, Eriochrome Blue Black B and Quinalizarin might be useful in differentiating between various types of Influenza versus Rhinovirus. Plasmocorinth B might also be suitable for a diagnosis of Parainfluenza.

## Claims

1. A method for rapidly detecting microorganisms in an upper respiratory test sample, the method comprising:
contacting a test strip with the upper respiratory test sample, the test strip comprising at least one broad spectrum indicator that exhibits a first spectral response in the presence of bacteria and a second spectral response in the presence of viruses, the test strip further comprising an array that contains at least one differentiating indicator, the array exhibiting a third spectral response in the presence of one type of microorganism and a fourth spectral response in the presence of another type of microorganism;
observing the broad spectrum indicator for the first spectral response or the second spectral response, the presence of the second spectral response indicating the presence of a virus in the sample; and
thereafter, observing the array for the third spectral response or the fourth spectral response;
wherein the broad spectrum indicator is a solvatochromatic indicator and wherein the differentiating indicator contains a pH-sensitive indicator, metal complexing indicator, or both.

2. The method of claim 1, wherein the solvatochromatic indicator is an N-phenolate betaine, in particular Reichardt's dye.

3. The method of claim 1 or 2, wherein the pH-sensitive indicator is a phthalein, hydroxyanthraquinone, arylmethane, aromatic azo, or a derivative thereof.

4. The method of any of the foregoing claims, wherein the metal complexing indicator is an aromatic azo compound.

5. The method of any of the foregoing claims, wherein the spectral responses are visually observed.

6. The method of any of the foregoing claims, wherein the array exhibits the third spectral response in the presence of gram-negative bacteria and the fourth spectral response in the presence of gram-positive bacteria.

7. The method of claim 6, wherein the gram-positive bacteria include *Streptococcus pyogenes, Streptococcus pneumoniae,* or a mixture thereof, and/or wherein the gram-negative bacteria include *Moraxella lacunata, Haemophilus influenzae, Chlamydia pneumoniae,* or a mixture thereof.

8. The method of any of the foregoing claims, wherein the array exhibits the fourth spectral response in the presence of one type of virus and the fifth spectral response in the presence of another type of virus.

9. The method of claim 8, wherein the array exhibits the fourth spectral response in the presence of Rhinoviruses.

10. The method of claim 9, wherein the array exhibits the fifth spectral response in the presence of Influenzavirus A, Influenzavirus B, Influenzavirus C, or combinations thereof.

11. The method of claim 10, wherein the array exhibits the fifth spectral response in the presence of Human Parainfluenza viruses.

12. The method of any of the foregoing claims, wherein the array contains from 2 to 50 individual array addresses, and preferably from 3 to 40 individual array addresses.

13. A kit for rapidly detecting microorganisms in an upper respiratory test sample, the kit comprising:
a device for collecting a test sample from an upper respiratory tract of a host; and
a test strip comprising at least one broad spectrum indicator that exhibits a first spectral response in the presence of bacteria and a second spectral response in the presence of viruses, the test strip further comprising an array that contains at least one differentiating indicator, the array exhibiting a third spectral response in the presence of one type of microorganism and a fourth spectral response in the presence of another type of microorganism;
wherein the broad spectrum indicator is a solvatochromatic indicator and wherein the differentiating indicator contains a pH-sensitive indicator, metal complexing indicator, or both.

14. The kit of claim 13, wherein the broad spectrum indicator is an N-phenolate betaine, in particular Reichardt's dye.

15. The kit of claim 13 or 14, wherein the device is a swab.

## Patentansprüche

1. Verfahren zum schnellen Detektieren von Mikroorganismen in einer Testprobe der oberen Atemwege, wobei das Verfahren umfasst:
Kontaktieren eines Teststreifens mit der Testprobe der oberen Atemwege, wobei der Teststreifen mindestens einen Indikator mit breitem Spektrum umfasst, der eine erste spektrale Antwort in Anwesenheit von Bakterien zeigt und eine zweite spektrale Antwort in Anwesenheit von Viren zeigt, wobei der Teststreifen des Weiteren ein Array umfasst, welches mindestens einen unterscheidenden Indikator enthält, wobei das Array eine dritte spektrale Antwort in Anwesenheit eines Typs von Mikroorganismen zeigt und eine vierte spektrale Antwort in Anwesenheit eines anderen Typs von Mikroorganismen zeigt;
Beobachten des Indikators mit breitem Spektrum hinsichtlich der ersten spektralen Antwort oder der zweiten spektralen Antwort, wobei das Vorhandensein der zweiten spektralen Antwort die Anwesenheit von einem Virus in der Probe anzeigt; und
danach, Beobachten des Arrays hinsichtlich der dritten spektralen Antwort oder der vierten spektralen Antwort;
wobei der Indikator mit breitem Spektrum ein solvatochromatischer Indikator ist und wobei der unterscheidende Indikator einen pH-empfindlichen Indikator, einen metallkomplexierenden Indikator oder beide enthält.

2. Verfahren nach Anspruch 1, wobei der solvatochromatische Indikator ein N-Phenolat-Betain ist, insbesondere Reichardts Farbstoff.

3. Verfahren nach Anspruch 1 oder 2, wobei der pH-empfindliche Indikator Phthalein, Hydroxyanthrachinon, Arylmethan, aromatisches Azo oder ein Derivat davon ist.

4. Verfahren nach einem der vorherigen Ansprüche, wobei der metallkomplexierende Indikator eine aromatische Azoverbindung ist.

5. Verfahren nach einem der vorherigen Ansprüche, wobei die spektralen Antworten visuell beobachtet werden.

6. Verfahren nach einem der vorherigen Ansprüche, wobei das Array die dritte spektrale Antwort in Anwesenheit von gramnegativen Bakterien zeigt und die vierte spektrale Antwort in Anwesenheit von grampositiven Bakterien zeigt.

7. Verfahren nach Anspruch 6, wobei die grampositiven Bakterien *Streptococcus pyogenes, Streptococcus pneumoniae* oder eine Mischung davon beinhalten, und/oder wobei die gramnegativen Bakterien *Moraxella lacunata, Haemophilus influenzae, Chlamydia pneumoniae* oder eine Mischung davon beinhalten.

8. Verfahren nach einem der vorherigen Ansprüche, wobei das Array die vierte spektrale Antwort in Anwesenheit eines Typs von Virus zeigt und die fünfte spektrale Antwort in Anwesenheit eines anderen Typs von Virus zeigt.

9. Verfahren nach Anspruch 8, wobei das Array die vierte spektrale Antwort in Anwesenheit von Rhinoviren zeigt.

10. Verfahren nach Anspruch 9, wobei das Array die fünfte spektrale Antwort in Anwesenheit von Influenzavirus A, Influenzavirus B, Influenzavirus C oder Kombinationen davon zeigt.

11. Verfahren nach Anspruch 10, wobei das Array die fünfte spektrale Antwort in Anwesenheit menschlicher Parainfluenzaviren zeigt.

12. Verfahren nach einem der vorherigen Ansprüche, wobei das Array von 2 bis 50 individuelle Arrayadressen enthält und vorzugsweise von 3 bis 40 individuelle Arrayadressen.

13. Kit zum schnellen Detektieren von Mikroorganismen in einer Testprobe der oberen Atemwege, wobei das Kit umfasst:
eine Vorrichtung zum Nehmen einer Testprobe aus einem oberen Atemwegstrakt eines Wirts; und
einen Teststreifen umfassend mindestens einen Indikator mit breitem Spektrum, der eine erste spektrale Antwort in Anwesenheit von Bakterien und eine zweite spektrale Antwort in Anwesenheit von Viren zeigt, wobei der Teststreifen des Weiteren ein Array umfasst, das mindestens einen unterscheidenden Indikator enthält, wobei das Array eine dritte spektrale Antwort in Anwesenheit eines Typs von Mikroorganismus zeigt und eine vierte spektrale Antwort in Anwesenheit eines anderen Typs von Mikroorganismus zeigt;
wobei der Indikator mit breitem Spektrum ein solvatochromatischer Indikator ist und wobei der unterscheidende Indikator einen pH-empfindlichen Indikator, einen metallkomplexierenden Indikator oder beide enthält.

14. Kit gemäß Anspruch 13, wobei der Indikator mit breitem Spektrum ein N-Phenolat-Betain ist, insbesondere Reichardts Farbstoff.

15. Kit gemäß Anspruch 13 oder 14, wobei die Vorrichtung ein Tupfer ist.

## Revendications

1. Procédé pour la détection rapide de micro-organismes dans un échantillon à tester issu des voies respiratoires supérieures, le procédé comprenant :
la mise en contact d'une bande test avec l'échantillon à tester issu des voies respiratoires supérieures, la bande test comprenant au moins un indicateur à large spectre qui produit une première réponse spectrale en présence de bactéries et une seconde réponse spectrale en présence de virus, la bande test comprenant en outre une matrice qui contient au moins un indicateur de différenciation, la matrice produisant une troisième réponse spectrale en présence d'un type de micro-organisme et une quatrième réponse spectrale en présence d'un autre type de micro-organisme ;
l'observation de l'indicateur à large spectre pour ce qui est de la première réponse spectrale ou de la seconde réponse spectrale, la présence de la seconde réponse spectrale indiquant la présence d'un virus dans l'échantillon ;
puis l'observation de la matrice pour ce qui est de la troisième réponse spectrale ou de la quatrième réponse spectrale,
procédé dans lequel l'indicateur à large spectre est un indicateur solvatochromique et l'indicateur de différenciation contient un indicateur sensible au pH, un indicateur de complexation métallique, ou les deux.

2. Procédé selon la revendication 1, dans lequel l'indicateur solvatochromique est une N-phénolate bétaïne, en particulier le colorant de Reichardt.

3. Procédé selon la revendication 1 ou 2, dans lequel l'indicateur sensible au pH est une phtaléine, une hydroxyanthraquinone, un arylméthane, un composé azo aromatique, ou un dérivé de ceux-ci.

4. Procédé selon l'une quelconque des revendications précédentes, dans lequel l'indicateur de complexation métallique est un composé azo aromatique.

5. Procédé selon l'une quelconque des revendications précédentes, dans lequel les réponses spectrales sont observées visuellement.

6. Procédé selon l'une quelconque des revendications précédentes, dans lequel la matrice produit la troisième réponse spectrale en présence de bactéries Gram négatives et la quatrième réponse spectrale en présence de bactéries Gram positives.

7. Procédé selon la revendication 6, dans lequel les bactéries Gram positives incluent *Streptococcus pyogenes, Streptococcus pneumoniae,* ou un mélange de ceux-ci et/ou les bactéries Gram négatives incluent *Moraxella lacunata, Haemophilus influenzae, Chlamydia pneumoniae,* ou un mélange de ceux-ci.

8. Procédé selon l'une quelconque des revendications précédentes, dans lequel la matrice produit la quatrième réponse spectrale en présence d'un type de virus et la cinquième réponse spectrale en présence d'un autre type de virus.

9. Procédé selon la revendication 8, dans lequel la matrice produit la quatrième réponse spectrale en présence de Rhinovirus.

10. Procédé selon la revendication 9, dans lequel la matrice produit la cinquième réponse spectrale en présence de *Influenzavirus A, Influenzavirus B, Influenzavirus C,* ou de leurs combinaisons.

11. Procédé selon la revendication 10, dans lequel la matrice produit la cinquième réponse spectrale en présence des virus Parainfluenza humains.

12. Procédé selon l'une quelconque des revendications précédentes, dans lequel la matrice contient de 2 à 5 adresses individuelles de matrice, et de préférence de 3 à 40 adresses individuelles de matrice.

13. Kit pour la détection rapide de micro-organismes dans un échantillon à tester issu des voies respiratoires supérieures, le kit comprenant :
un dispositif pour collecter un échantillon à tester depuis les voies respiratoires supérieures d'un hôte ; et
une bande test comprenant au moins un indicateur à large spectre qui produit une première réponse spectrale en présence de bactéries et une seconde réponse spectrale en présence de virus, la bande test comprenant en outre une matrice qui contient au moins un indicateur de différenciation, la matrice produisant une troisième réponse spectrale en présence d'un type de micro-organisme et une quatrième réponse spectrale en présence d'un autre type de micro-organisme,
l'indicateur à large spectre étant un indicateur solvatochromique et l'indicateur de différenciation contenant un indicateur sensible au pH, un indicateur de complexation métallique, ou les deux.

14. Kit selon la revendication 13, dans lequel l'indicateur à large spectre est une N-phénolate bétaïne, en particulier le colorant de Reichardt.

15. Kit selon la revendication 13 ou 14, dans lequel le dispositif est un écouvillon.
